(19) Europäisches Patentamt / European Patent Office / Office européen des brevets

(11) **EP 2 239 269 B1**

## (12) EUROPEAN PATENT SPECIFICATION

(45) Date of publication and mention
of the grant of the patent:
**16.01.2013 Bulletin 2013/03**

(51) Int Cl.:
***C07K 14/00*** *(2006.01)*     ***A61K 38/16*** *(2006.01)*
***G01N 33/53*** *(2006.01)*

(21) Application number: **10171553.0**

(22) Date of filing: **24.09.1999**

(54) **Use of peptides derived from copolymer-1 as molecular weight markers**

Verwendung von mit Copolymer-1 verwandten Peptiden als Molekulargewichtsmarker

Utilisation de peptides associés au copolymère 1 en tant que marqueurs de poids moléculaire

(84) Designated Contracting States:
**AT BE CH CY DE DK ES FI FR GB GR IE IT LI LU MC NL PT SE**

(30) Priority: **25.09.1998 US 101693 P**

(43) Date of publication of application:
**13.10.2010 Bulletin 2010/41**

(62) Document number(s) of the earlier application(s) in accordance with Art. 76 EPC:
**09004306.8 / 2 090 583**
**99949923.9 / 1 115 743**

(73) Proprietor: **YEDA RESEARCH AND DEVELOPMENT CO., LTD.**
**76100 Rehovot (IL)**

(72) Inventors:
• **Gad, Alexander**
**74061 Nes Ziona (IL)**
• **Lis, Dora**
**38000 Hadera (IL)**

(74) Representative: **Vossius & Partner**
**Siebertstrasse 4**
**81675 München (DE)**

(56) References cited:
**WO-A1-95/31990**

• **VACCINE., vol. 10, no. 14, 1992, pages 991-999, XP002318646 GB BUTTERWORTH SCIENTIFIC. GUILDFORD.**
• **D TEITELBAUM ET AL.: "Copolymer 1 from the laboratory to the FDA" ISRAEL JOURNAL OF MEDICAL SCIENCES., vol. 33, 1997, pages 280-284, XP2924839 ISRAEL MEDICAL ASSOCIATION, TEL AVIV. ISSN: 0021-2180**

**Description**

INTRODUCTION

The present invention provides molecular weight markers for accurate determination of the molecular weigh of glatiramer acetate, terpolymers and other copolymers. The molecular weight markers are polypeptides having identified molecular weights between about 2,000 daltons and about 40,000 daltons, and an amino acid composition corresponding to glatiramer acetate or a rotated copolymer. Identified molecular weights are provided by polypeptides having defined sequences. Molecular weight markers corresponding to glatiramer acetate comprise the amino acids alanine, glutamic acid, tyrosine and lysine in specific molar ratios. Molecular weight markers corresponding to related terpolymers comprises three of the four amino acids. In a preferred embodiment, the polypeptide has alanine at the N-terminus and tyrosine at the fourth position from the N-terminus. The present invention further provides a plurality of molecular weight markers for determining the molecular weight range of a copolymer composition. The plurality of molecular weight markers ideally displays linear relationships between molar ellipticity and molecular weight, or between retention time and the log of molecular weight.

Optimally, the polypeptides demonstrate biological activity similar to the copolymer from which they are derived. Polypeptides having defined molecular weights and amino acid compositions similar to glatiramer acetate optimally have therapeutic utility for the treatment of immune diseases and conditions.

BACKGROUND OF THE INVENTION

Autoimmune diseases occur when an organism's immune system fails to recognize some of the organism's own tissues as "self" and attacks them as foreign." Normally, self-tolerance is developed early by developmental events within tne immune system that prevent the organism's own T cells and B cells from reacting with the organism's own tissues. These early immune responses are mediated by the binding of antigens to MHC molecules and presentation to T cell receptors.

This self-tolerance process breaks down when autoimmune diseases develop and the organism's own tissues and proteins are recognized as "autoantigens" and attacked by the organism's immune system. For example, multiple sclerosis is believed to be an autoimmune disease occurring when the immune system attacks the myelin sheath, whose function is to insulate and protect nerves. It is a progressive disease characterized by demyelination, followed by neuronal and motor function loss. Rheumatoid arthritis ("RA") is also believed to be an autoimmune disease which involves chronic inflammation of the synovial joints and infiltration by activated T cells, macrophages and plasma cells, leading to a progressive destruction of the articular cartilage. It is the most severe form of joint disease. The nature of the autoantigen (s) attacked in rheumatoid arthritis is poorly understood, although collagen type II is a candidate.

A tendency to develop multiple sclerosis and rheumatoid arthritis is inherited. These diseases occur more frequently in individuals carrying one or more characteristic MHC class II alleles. For example, inherited susceptibility for rheumatoid arthritis is strongly associated with the MHC class II DRB1 *0401, DRB 1 *0404, or DRB 1*0405 or the DRB1*0101 alleles. The histocompatibility locus antigens (HLA) are found on the surface of cells and help determine the individuality of tissues from different persons. Genes for histocompatibility locus antigens.are located in the same region of chromosome 6 as the major histocompatibility complex (MHC). The MHC region expresses a number of distinctive classes of molecules in various cells of the body, the genes being, in order of sequence along the chromosome, the Class I, II and III MHC genes. The Class I genes consist of HLA genes, which are further subdivided into A, B and C subregions. The Class II genes are subdivided into the DR, DQ and DP subregions. The MHC-CR molecules are the best known; these occur on the surfaces of antigen presenting cells such as macrophages, dendritic cells of lymphoid tissue and epidermal cells. The Class III MHC products are expressed in various components of the complement system, as well as in some non-immune related cells.

A number of therapeutic agents have been developed to treat autoimmune diseases, including steroidal and non-steroidal anti-inflammatory drugs, for example, methotrexate; various interferons; and certain inhibitors of prostaglandin synthesis. However, these agents can be toxic when used for more than short periods of time or cause undesirable side effects. Other therapeutic agents bind to and/or inhibit the inflammatory activity of tumor necrosis factor (TNF), for example, anti-TNF specific antibodies or antibody fragments, or a soluble form of the TNF receptor. These agents target a protein on the surface of a T cell and generally prevent interaction with an antigen presenting cell (APC). However, therapeutic compositions containing natural folded proteins are often difficult to produce, formulate, store, and deliver. Moreover, the innate heterogeneity of the immune system can limit the effectiveness of drugs and complicate long-term treatment of autoimmune diseases.

Glatiramer acetate (Copolymer 1; Cop 1; hereinafter GLAT copolymer) is a mixture of polypeptides composed of alanine, glutamic acid, lysine, and tyrosine in a molar ratio of approximately 4.6:1.5:3.0:1.0, respectively, which is synthesized by chemically polymerizing the four amino acids, forming products with average molecular weights ranging from about 4000 to about 13,000 daltons. The corresponding molar fractions are approximately 0.427 for alanine, 0.141 for glutamic acid, 0.337 for lysine and 0.093 for tyrosine, and may vary by about +/-10%. Related copolymers are mixtures of polypeptides composed of three (thus, "terpolymers") of the four aforementioned amino acids. Copolymer 1 and the terpolymers address the innate heterogeneity of the mammalian immune system and human population and are effective for treatment of autoimmune diseases and other immune conditions. Preferred average molecular weight ranges and processes of making terpolymers are described in U,S, Patent No. 5,800,808. Also contemplated by the invention are other copolymers comprised of other combinations of three, four, or five or more amino acids.

To certify a Copolymer 1 or terpolymer preparation for use in a pharmaceutical products, it is necessary to accurately determine the molecular weight distribution of the polypeptides in the preparation. One method for determining the molecular weight is chromatography on a Superose 12 column. Calibration coefficients of columns for determination of glatiramer acetate molecular weight have been determined using glatiramer acetate batches with indirectly measured molecular weights. Indirect measures have included viscosimetry and velocity-sedimentation ultracentrifugation. More recently, batches of glatiramer acetate markers have been prepared whose molecular weights were determined by multiple angle laser light scattering (MALLS).

Thus, a need exists for molecular weight markers useful as standards for determining the molecular weight distribution of copolymer compositions contemplated by the invention. Desirable molecular weight markers have defined molecular weights and physical properties which are analogous to the molecules for which molecular weight is to be determined, ideally, there is a linear relationship between the defined mo!ecular weights (or the log of the defined molecular weights) of the markers and a measurable physical property such as, for example, the molar ellipticity of the markers, or the retention time of the markers on a molecular sizing column.

Isr. J Med. Sci, 33, pages 280-286 (1997) discloses copolymer 1 for the treatment of multiple sclerosis (MS) and experimental allergic encephalomyelitis, an animal model of MS.

## SUMMARY OF THE INVENTION

Sequence-defined molecular weight markers that have chemical and physical characteristics similar to GLAT copolymer provide an accurate and robust calibration set for determinations of molecular of production batches. The present invention provides derivatives of GLAT copolymer useful as molecular weight markers for determining the molecular weight ranges of GLAT copolymer preparations and optimally having therapeutic utility for treatment of immune conditions. The invention further provides polypeptides having defined molecular weights which are derivatives of other copolymers and which are useful for determining molecular weight ranges of preparations of those copolymers. When those copolymers are therapeutically useful, the derivative polypeptides optimally have therapeutic utility. For determination of the molecular weight range of a GLAT copolymer preparation, the preferred derivative is a polypeptide having an amino acid compositon corresponding approximatety to GLAT copolymer and an identified molecular weight which is between about 2,000 daltons and about 40,000 daltons. The polypeptide preferably has specific molar ratios of amino acids alanine, glutamic acid, and lysine. Moreover, in a preferred embodiment the polypeptide has alanine at the N-terminus and tyrosine at the fourth position from the N-terminus. For determination of the molecular weight of a terpolymer, the preferred derivative will have a defined molecular weight and an amino acid composition corresponding approximately to that of the terpolymer. Other copolymers are also contemplated by the invention. When determining of the molecular weight of a copolymer contemplated by the invention, the polypeptide derivative will have a defined molecular weight and an amino acid composition corresponding approximately to that of the copolymer.

The present invention further provides a plurality of molecular weight markers for determining the molecular weight of glatiramer acetate or a terpolymer or other copolymer or a molecular weight sizing column. The markers comprise two to ten or more polypeptides, each polypeptide having an identified molecular weight. When determining the molecular weight range of glatiramer acetate, a preferred plurality of molecular weight markers will have defined molecular weights from about 2,000 daltons to about 40,000 daltons, and amino acid compositions corresponding to glatiramer acetate or a selected terpolymer. In preferred embodiments, there is a linear relationship between the log molecular weight of the polypeptide molecular weight markers and either the retention time of the molecular weight markers on a sizing column or between the molecular weight of the molecular weight markers and the molar ellipticity of the molecular weight markers.

The present invention also provides a process for preparing a pharmaceutical product comprising glatiramer acetate having an average molecular weight from 4,000 to 13,000 Daltons which process comprises the steps of obtaining a glatiramer acetate preparation; determining the average molecular weight of the glatiramer acetate preparation using a chromatographic apparatus which is calibrated using a plurality of molecular weight markers to establish a linear relationship between the retention time of the molecular weight markers on the chromatographic apparatus and the log of the molecular weight of the molecular weight markers, wherein each of the molecular weight markers is a polypeptide consisting of alanine, glutamic acid, tyrosine and lysine and having a predetermined amino acid sequence and defined molecular weight; and using the glatiramer acetate preparation to prepare the pharmaceutical product if the glatiramer acetate preparation has an average molecular weight from 4,000 to 13,000 Daltons.

The present invention also provides a process for certifying glatiramer acetate for use in a pharmaceutical product comprising glatiramer acetate having an average molecular weight from 4,000 to 13,000 Daltons which process comprises the steps of determining the average molecular weight of the glatiramer acetate using a chromatographic apparatus which is calibrated using a plurality of molecular weight markers to establish a linear relationship between the retention time of the molecular weight markers on the chromatographic apparatus and the log of the molecular weight of the molecular weight markers, wherein each of the molecular weight markers is a polypeptide consisting of alanine, glutamic acid, tyrosine and lysine and having a predetermined amino acid sequence and defined molecular weight; and certifying the glatiramer acetate for use in the pharmaceutical product if the glatiramer acetate has an average molecular weight from 4,000 to 13,000 Daltons.

In one or more embodiments, in the molecular weight markers the molar fraction of alanine is 0.38 to 0.5, of glutamic acid is 0.13 to 0.15, of tyrosine is 0.08 to 0.10 and of lysine is 0.3 to 0.4.

In one or more embodiments, in the molecular weight markers the molar fraction of alanine is 0.422 to 0.444, of glutamic acid is 0.133 to 0.143, of tyrosine is 0.086 to 0.093 and of lysine is 0.333 to 0.349.

In one or more embodiments, the chromatographic apparatus is a gel permeation chromatography column.

In one or more embodiments, wherein the chromatographic apparatus is a TSK column, a Sephadex column, a Sepharose column, or a Superose column.

In one or more embodiments, one of the molecular weight markers is:

AKKYAKKEKAAKKAYf:KEAKAKAAEAAAKEAAYEA (SEQ ID NO: 1);

AKKYAKKAKAEKAKKAYKAAEAKKAAKYEKAAAEKAAAKEAAYEA (SEQ ID NO: 2);

AKKYAKKEKAYAKKAEKAAKKAEAKAYKAAEAKKKAEAKYKAEAAKAAAKEAAYEA (SEQ ID NO: 3);

AKKYAKKEKAYAKAKKAEAKAAKKAKAEAKKYAKAAKAEKKEYAAAEAKYKAEAAKAAAKEA AYEA (SEQ ID NO: 4);

AKKYAKKEKAYAKKAEKAAKKAEAKAYKAAEAKKKAKAEAKKYAKAAKAEKKEYAAAEAKYK AEAAKAAAKEAAYEA (SEQ ID NO: 5);

AKKYAKKEKAYAKKAEKAAKKAEAKAYKAAEAKKKAKAEAKKYAKAAKAEKKEYAAAEAKYK AEAAKKAYKAEAAKAAAKEAAYEA (SEQ ID NO: 6); or

AKKYAKKAEKAYAKKAKAAKEKKAYAKKEAKAYKAAEAKKKAKAEAKKYAKEAAKAKKEAYK AEAKKYAKAAKAEKKEYAAAEAKKAEAAKAYKAEAAKA AAKEAAYEA (SEQ ID NO: 7),

wherein A represents alanine, K represents lysine, Y represents tyrosine, and E represents glutamic acid.

In one or more embodiments, the plurality of molecular weight markers is:

AKKYAKKEKAAKKAYKKEAKAKAAEAAAKEAAYEA (SEQ ID NO: 1);

AKKYAKKAKAEKAKKAYKAAEAKKAAKYEKAAAEKAAAKEAAYEA (SEQ ID NO: 2);

AKKYAKKEKAYAKKAEKAAKKAEAKAYKAAEAKKKAEAKYKAEAAKAAAKEAAYEA (SEQ ID NO: 3);

AKKYAKKEKAYAKAKKAEAKAAKKAKAEAKKYAKAAKAEKKEYAAAEAKYKAEAAKAAAKEA AYEA (SEQ ID NO: 4);

AKKYAKKEKAYAKKAEKAAKKAEAKAYKAAEAKKKAKAEAKKYAKAAKAEKKEYAAAEAKYK
AEAAKAAAKEAAYEA (SEQ ID NO: 5);

AKKYAKKEKAYAKKAEKAAKKAEAKAYKAAEAKKKAKAEAKKYAKAAKAEKKEYAAAEAKYK
AEAAKKAYKAEAAKAAAKEAAYEA (SEQ ID NO: 6); and

AKKYAKKAEKAYAKKAKAAKEKKAYAKKEAKAYKAAEAKKKAKAEAKKYAKEAAKAKKEAYK
AEAKKYAKAAKAEKKEYAAAEAKKAEAAKAYKAEAAKA AAKEAAYEA (SEQ ID NO: 7),

wherein A represents alanine, K represents lysine, Y represents tyrosine, and E represents glutamic acid.

BRIEF DESCRIPTION OF THE DRAWINGS

Figure 1a provides the distribution of alanine in the molecular markers (TV-markers) described in Table 1. The amino acid position is defined by the X-axis. The presence of an alanine is indicated by a vertical bar at the indicated amino acid position.

Figure 1b provides the distribution of lysine in the TV-markers described in Table 1. The amino acid position is defined by the X-axis, The presence of a lysine residue is indicated by a vertical bar at the indicated amino acid position.

Figure 1c provides the distribution of glutamic acid in the TV-markers described in Table 1. The amino acid position is defined by the X-axis. The presence of a glutamic acid residue is indicated by a vertical bar at the indicated amino acid position.

Figure 1d provides the distribution of tyrosine in the TV-markers described in Table 1. The amino acid position is defined by the X-axis. The presence of a tyrosine, residue is indicated by a vertical bar at the indicated amino acid position.

Figure 2 provides a plot of the molar ellipticity versus molecular weight of the present TV-markers compared to known glatiramer acetate markers. The molar ellipticity is provided in $10^{-5}$ deg cm$^{-2}$ dmole$^{-1}$ and the molecular weight is in daltons. Circies indicate TV-markers and squares depict glatiramer acetate markers. As shown, the TV-markers provide a linear relationship between molar ellipticity and molecular weight.

Figure 3a provides a plot of the relative retention time (RRT) of the present TV-markers versus the log molecular weight of those markers, using the RRT-based algorithm.

Figure 3b provides a plot of the log molecular weight of the TV-markers versus the retention time (RT) of those markers, using the Millennium-based algorithm.

Figure 4a provides a plot summarizing several calibrations of the relative retention time (RRT) of the present TV-markers versus the molecular weight of those markers, using the RRT-based algorithm. Data were obtained from sixteen columns. Average values for each of the sixteen calibrations are depicted.

Figure 4b provides a plot summarizing several calibrations of the molecular weight of the TV-markers versus the relative retention time (RRT) of those markers, using the Millennium-based algorithm. Data were obtained from sixteen columns. Average values for each of the sixteen calibrations are depicted.

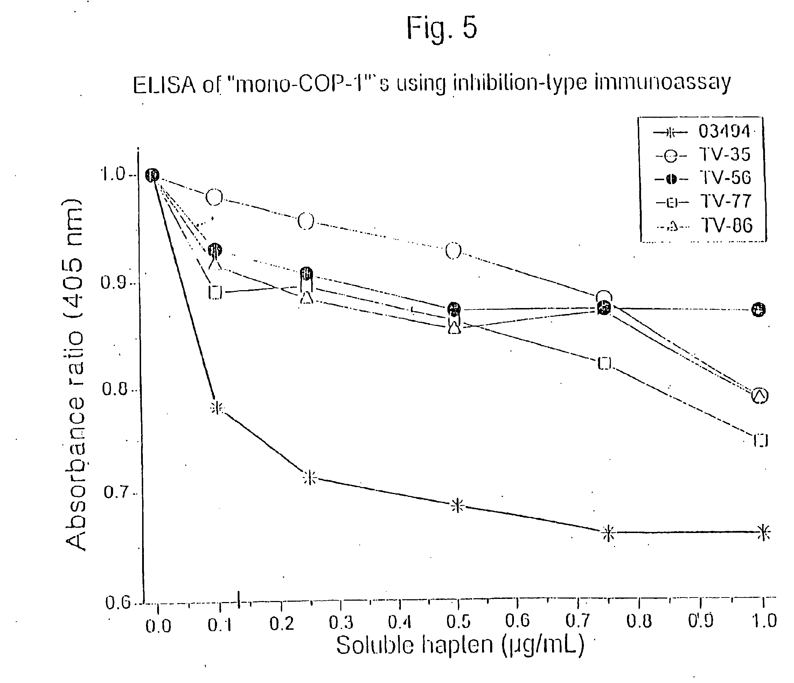

Figure 5 depicts inhibition of Cop 1 binding to anti-Cop 1 polyclonal antibodies by four TV-markers and Cop 1 (03494). Absorbance ratio indicates absorbance measured with increasing inhibitor concentration relative to absorbance in the absence of binding inhibition. Figure 5 is for comparison.

DETAILED DESCRIPTION OF THE INVENTION

Molecular weight markers of the invention (e.g., TV-markers), include polypeptides having an amino acid composition approximately corresponding to glatiramer acetate or related terpolymers, and an identified molecular weight which is between about 2,000 daltons and about 40,000 daltons and are useful for accurately determining the molecular weight of GLAT copolymer and related terpolymers. It follows from the requirement for an identified molecular weight that a TV-marker should have a discrete molecular weight and not a range of molecular weights. Accordingly, TV-markers are synthesized according to a predetermined amino acid sequence which corresponds in composition to the copolymer for which molecular weight range is to be determined. Optimally, TV-markers have therapeutic activity which is similar to corresponding copolymer. These markers can be used in any molecular size discrimination system using any available molecular weight determination procedure or apparatus. For example, the present markers can be used for calibration of any chromatographic procedure or apparatus which is used for molecular weight determinations of polypeptides or proteins. Such a chromatographic apparatus can be a molecular weight sizing column which separates polypeptides on the basis of their molecular size. Examples of molecular weight sizing columns include TSK columns, Sephadex columns, Sepharose columns, and Superose columns. In order to provide molecular weight markers of discrete size and composition, molecular weight markers of the invention can be synthesized according to predetermined sequences by methods which are well known to those of skill in the art.

The phrases "amino acid" and "amino acid sequence" as defined here and in the claims can include one or more components which are amino acid derivatives and/or amino acid analogs comprising part or the entirety of the residues for any one or more of the 20 naturally occurring amino acids indicated by that sequence. For example, in an amino acid sequence having one or more tyrosine residues, a portion of one or more of those residues can be substituted with homotyrosine. Further, an amino acid sequence having one or more non-peptide or peptidomimetic bonds between two adjacent residues, is included within this definition.

The one letter and three letter amino acid codes (and the amino acid that each represents) are as follows: A means ala (alanine); C means cys (cysteine); D means asp (aspartic acid); E means glu (glutamic acid); F means phe (phenylalanine); G means gly (glycine); H means his (histidine); 1 means ile (isoleucine); K means lys (lysine); L means leu (leucine); M means met (methionine); N means asn (asparagine); P means pro (proline); Q means gln (glutamine); R means arg (arginine); S means ser (serine); T means thr (threonine); V means val (valine); W means trp (tryptophan); and Y means tyr (tyrosine).

The term "hydrophobic" amino acid is defined here and in the claims as including aliphatic amino acids alanine (A, or ala), glycine (G, or gly), isoleucine (I, or ile), leucine (L, or leu), proiine (P, or pro), and vaiine (V, or val), the terms in parentheses being the one letter and three letter standard code abbreviation s for each amino acid, and aromatic amino acids tryptophan (W, or trp), phenylalanine (F or phe), and tyrosine (Y, or tyr). The amino acids confer hydrophobicity as a function of the length of aliphatic and size of aromatic side chains, when found as residues within a protein.

The term "charged" amino acid is defined here and in the claims as including an amino acids aspartic acid (D, or asp), glutamic acid (E, or glu), histidine (H, or his), arginine (R, or arg) and lysine (K, or lys), which confer a positive (his, lys and arg) or negative (asp and gly) charge at physiological values of pH in aqueous solutions on proteins containing these residues.

Polypeptide Compositions Contemplated by the Invention - According to the present invention, polypeptides having defined molecular weights and comprising three or all four of the amino acids tyrosine, glutamic acid, alanine and lysine are preferred for the present markers.

Moreover, molecular weight markers of the invention can be composed of *L*- or *D*-amino acids. As is known by one of skill in the art, *L*-amino acids occur in most natural proteins. However, *D*-amino acids are commercially available and can be substituted for some or all of the amino acids used to make molecular weight markers of the invention. The present invention contemplates molecular weight markers formed from mixtures of *D*- and *L*-amino acids, as well as molecular weight markers consisting essentially of either *L*- or *D*-amino acids.

The average molecular weight and the average molar fraction of the amino acids in the present polypeptides can vary. However, a moiecuiar weight range of about 2,000 to about 40,000 is contemplated, and basic polypeptides, rather than acidic polypeptides, are preferred.

In one embodiment, the present invention provides polypeptide markers containing tyrosine, alanine, glutamic acid and lysine in defined molar ratios. In a more preferred embodiment, the molar ratio of amino acids of the present polypeptides is that found in GLAT copolymer. Such a correspondence in molar ratios providers the best molecular weight markers because those markers will have a charge and a molecular shape which is similar to that of GLAT copolymer. When structurally dissimilar markers are used, the markers may migrate or elute somewhat differently from GLAT copolymer preparations, even though those preparations have the same molecular weight as the markers.

Moreover, in a preferred embodiment, alanine is at the N-terminus and tyrosine is at position four from the N-terminus. Edman degradation analyses performed on various glatiramer acetate batches revealed a greater abundance of alanine at the N-terminus and tyrosine at position four from the N-terminus. Therefore, in certain preferred embodiments, GLAT copolymer molecular weight markers have alanine at the N-terminus and tyrosine at position four from the N-terminus. Studies of the polymerization reaction used to synthesize GLAT copolymer have indicated that alanine and glutamic acid polymerize faster than lysine. As a result, the C-terminal portion of GLAT copolymer tends to be richer in alanine and glutamic acid, whereas the N-terminal portion tends to be richer in lysine. In preferred embodiments, the distribution of amino acid residues in GLAT copolymer molecular weight markers reflects this bias.

When determining the molecular weight range of GLAT copolymer, a preferred molecular weight marker consists essentially of amino acids alanine, glutamic acid, tyrosine and lysine in molar fractions of from about 0.38 to about 0.50 alanine, from about 0.13 to about 0.15 glutamic acid, from about 0.08 to about 0.10 tyrosine, and from about 0.3 to about 0.4 lysine.

In other embodiments, the present invention provides molecular weight markers containing three of the four amino acids alanine, glutamic acid, tyrosine, and lysine in defined ratios. In preferred embodiments, the molar fractions of amino acids present the molecular weight markers correspond to that found in a corresponding terpolymer.

When the molecular weight marker contains alanine, glutamic acid and tyrosine, alanine can be present in a mole fraction of about 0.005 to about 0.800, glutamic acid can be present in a mole fraction of about 0.005 to about 0.300, and tyrosine can be present in a mole fraction of about 0.005 to about 0.250. The molecular weight is from about 2,000 to about 40,000 daltons, and preferably from about 3000 to about 12,000 daltons.

When the molecular weight marker contains alanine, glutamic acid and lysine, alanine can be present in a mole fraction of about 0.005 to about 0.600, glutamic acid can be present in a mole fraction of about 0.005 to about 0.300, and lysine can be present in a mole fraction of about 0.2 to about 0.7. The molecular weight is between about 2,000 and about 40,000 daltons, and preferably between about 3000 and about 12,000 daltons.

When the molecular weight marker contains alanine, tyrosine and lysine, alanine can be present in a mole fraction of about 0.3 to about 0.6, tyrosine can be present in a mole fraction of about 0.005 to about 0.250, and lysine can be present in a mole fraction of about 0.1 to about 0.5. The molecular weight is between about 2.000 and about 40,000 daltons, and preferably between about 3000 and about 12,000 daltons.

When the molecular weight marker contains glutamic acid, tyrosine and lysine, glutamic acid can be present in a mole fraction of about 0.005 to about 0.300, tyrosine can be present in a mole fraction of about 0.005 to about 0.250, and lysine can be present in a mole fraction of about 0.3 to about 0.7. The molecular weight is between about 2,000 and about 40,000 daltons, and preferably between about 3000 and about 12,000 daltons.

Polypeptides of the invention can be used for molecular weight range determinations of other copolymers contemplated by the invention. Contemplated copolymers can consist of combinations of three, four, or five or more amino acids. In general, in order to determine the molecular weight range of a copolymer contemplated by the invention, the polypeptide molecular weight marker will have a defined molecular weight and an amino acid composition corresponding approximately to that of the copolymer. It will be apparent to one of skill in the art that any bias in the distribution of amino acids in a copolymer can be determined as described above for GLAT copolymer. For example, the relative amounts of amino acids incorporated at each position of a terpolymer population can be obtained by analyzing the products of each step of an Edman degradation. Alternatively, the proportions of amino acids incorporated into a terpolymer population during synthesis can be monitored. Where applicable, molecular weight markers can then be synthesized which reflect the bias. In addition, certain preferred terpolymer molecular weight markers will have alanine or tyrosine at position four.

Examples of preferred polypeptide molecular weight marker sequences are given in Table 1 (SEQ ID NOS: 1-7) using the conventional single letter amine acid code and reading from N-terminal to C-terminal, The seven indicated sequences are individual preparations of polypeptides having an amino acid composition corresponding to glatiramer acetate. Usually, amino acids comprising a molecular weight marker molecule are predominantly of one configuration (D- or L- configuration). In preferred embodiments, a molecular weight marker molecule is composed entirely of amino acids of the same configuration. However, molecular weight marker molecules comprising amino acids of mixed configuration may be preferred in certain embodiments where molecular weight is being determined for a glatiramer acetate preparation comprising amino acids of mixed configuration.

**Table 1. Selected TV-markers amino acid sequences**

| SEQ ID NO | Sequence - |
|---|---|
| 1 | AKKYAKKEKAAKKAYKKEAKAKAAEAAAKEAAYEA |
| 2 | AKKYAKKAKAEKAKKAYKAAEAKKAAKYEKAAAEKAAAKE-AAYEA |
| 3 | AKKYAKKEKAYAKKAEKAAKKAEAKAYKAAEAKKKAEAKY-KAEAAKAAAKEAAYEA |
| 4 | AKKYAKKEKAYAKAKKAEAKAAKKAKAEAKKYAKAAKAEK-KEYAAAEAKYKAEAAKAAAKEAAYEA |
| 5 | AKKYAKKEKAYAKKAEKAAKKAEAKAYKAAEAKKKAKAEA-KKYAKAAKAEKKEYAAAEAKYKAEAAKAAAKEAAYEA |

(continued)

| SEQ ID NO | Sequence - |
|---|---|
| 6 | AKKYAKKEKAYAKKAEKAAKKAEAKAYKAAEAKKKAKAEA-KKYAKAAKAEKKEYAAAEAKYKAEAAKKAYKAEAAKAAAK-EAAYEA |
| 7 | AKKYAKKAEKAYAKKAKAAKEKKAYAKKEAKAYKAAEAKK-KAKAEAKKYAKEAAKAKKEAYKAEAKKYAKAAKAEKKEYA-AAEAKKAEAAKAYKAEAAKAAAKEAAYEA |

In another embodiment, the present invention provides a plurality of molecular weight markers for determining the molecular weight of glatiramer acetate or a terpolymer on a molecular weight sizing column. The plurality of molecular weight markers are polypeptides. The plurality of markers can be two to about ten or more. In a preferred embodiment, the plurality of markers is about seven. Each polypeptide has an identified molecular weight which is between about 2.000 daltons and about 40,000 daltons, and an amino acid composition which corresponds approximately to that of glatiramer acetate or a terpolymer.

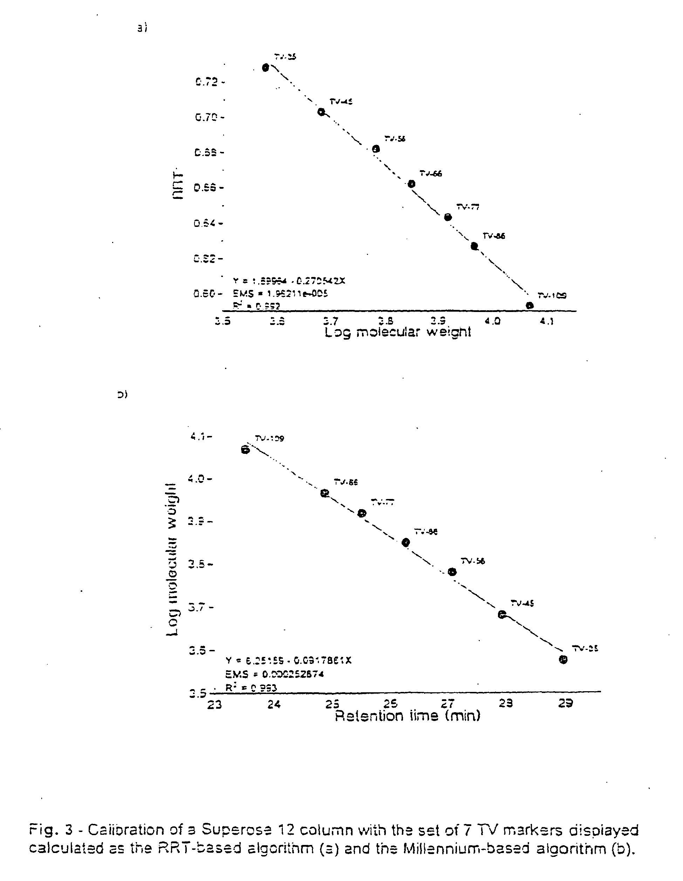

When such a plurality of molecular weight markers are used as standards for determining the molecular weight of glatiramer acetate or a terpolymer, a relationship which is approximately linear exists between the retention time of the molecular weight markers on the chromatographic column and the log of the molecular weight. A plurality of markers is used which is sufficient to establish the approximately linear relationship, although more may be employed. Fig. 3 shows the approximately linear relationship behveen relative retention time and log molecular weight for TV-markers of the invention.

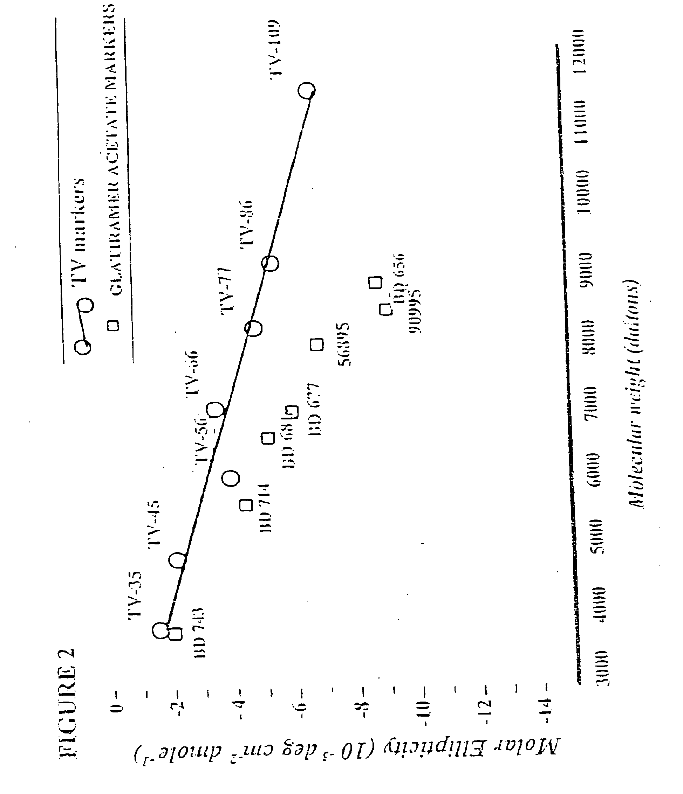

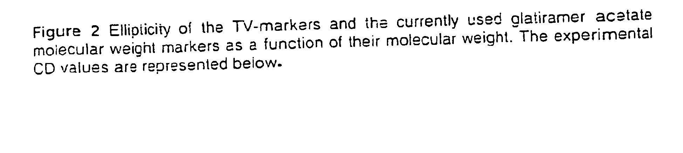

In another embodiment, an approximately linear relationship exists between the molar ellipticity of the molecular weight markers and the molecular weight of the markers. When determining the molecular weight of a glatiramer acetate preparation by molar ellipticity, a plurality of markers is used which is sufficient to establish the approximately linear relationship, although more may be employed. A molecular weight for the glatiramer acetate or terpolymer preparation is then obtained based on the linear relationship. Fig. 2 shows the approximately linear relationship between molar ellipticity and molecular weight for TV-markers of the invention.

The examples which follow describe the invention in detail with respect to showing how certain specific representative embodiments thereof can be made, the materials, apparatus and process steps being understood as examples that are intended to be illustrative only.

Throughout this application, various publications, patents, and patent applications have been referred to. The teachings and disclosures of these publications, patents, and patent applications in their entireties more fully describe the state of the art to which the present invention pertains.

The following examples further illustrate the invention.

## EXAMPLE 1

**Physical Properties of TV-markers**

**Solid phase synthesis**

Seven molecular weight markers were made with molecular weights ranging from about 3700-12000 daltons in the laboratory of Prof. M. Fridkin (Welzmann Institute of Science) (Table 2). These markers are referred to as TV-markers. The individual peptides were assigned a name TV-##, where ## is the number of amino acid residues (e.g. TV-35 is the 35-mer marker). The amino acid composition of these markers meets glatiramer acetate specifications (Table 2).

**Table 2**

TV-35 - Peptide with a molecular weight = 3757 daltons

| | Ala | Glu | Tyr | Lys |
|---|---|---|---|---|
| Number of residues | 15 | 5 | 3 | 12 |
| Molar fraction | 0.429 | 0.143 | 0.086 | 0.343 |

(continued)

| TV-45 - Peptide with molecular weight = 4790 daltons | | | | |
|---|---|---|---|---|
| | **Ala** | **Glu** | **Tyr** | **Lys** |
| Number of residues | 20 | 5 | 4 | 15 |
| Molar fraction | 0.444 | 0.133 | 0.089 | 0.333 |
| TV-56 - Peptide with a molecular weight = 6008 daltons | | | | |
| | **Ala** | **Glu** | **Tyr** | **Lys** |
| Number of residues | 24 | 8 | 5 | 19 |
| Molar fraction | 0.429 | 0.143 | 0.089 | 0.339 |
| TV-65 - Peptide with a molecular weight = 7040 daltons | | | | |
| | **Ala** | **Glu** | **Tyr** | **Lys** |
| Number of residues | 29 | 9 | 6 | 22 |
| Molar fraction | 0.439 | 0.136 | 0.091 | 0.333 |
| TV-77 - Peptide with a molecular weight = 8259 daltons | | | | |
| | **Ala** | **Glu** | **Tyr** | **Lys** |
| Number of residues | 33 | 11 | 7 | 25 |
| Molar fraction | 0.429 | 0.143 | 0.091 | 0.338 |
| TV-85 - Peptide with a molecular weight = 9220 daltons | | | | |
| | **Ala** | **Glu** | **Tyr** | **Lys** |
| Number of residues | 37 | 12 | 8 | 29 |
| Molar fraction | 0.430 | 0.140 | 0.093 | 0.337 |
| TV-109 - Peptide with a molecular weight = 11727 daltons | | | | |
| | **Ala** | **Glu** | **Tyr** | **Lys** |
| Number of residues | 46 | 15 | 10 | 38 |
| Molar fraction | 0.422 | 0.138 | 0.092 | 0.349 |

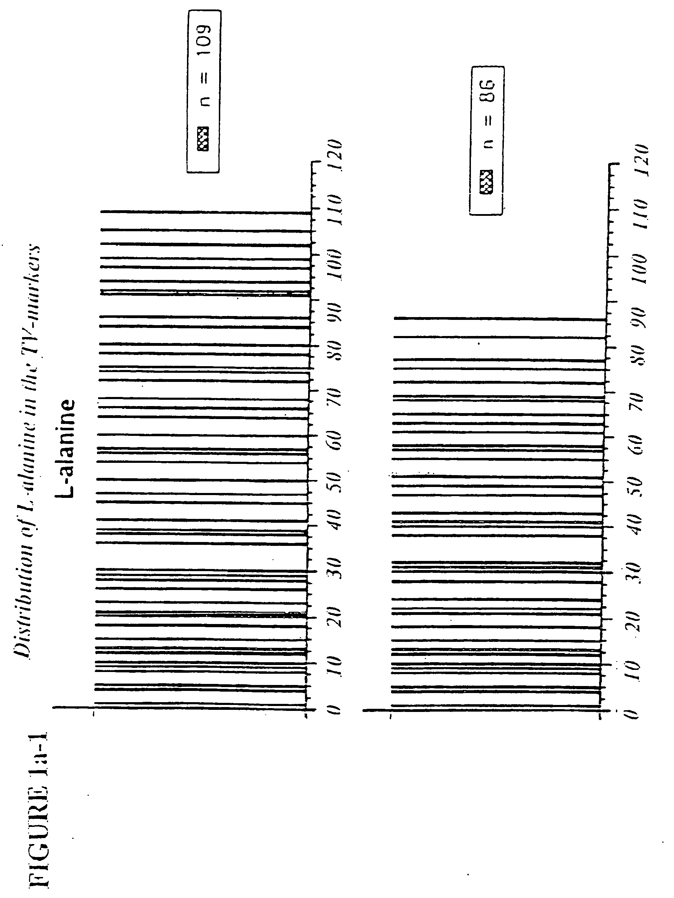

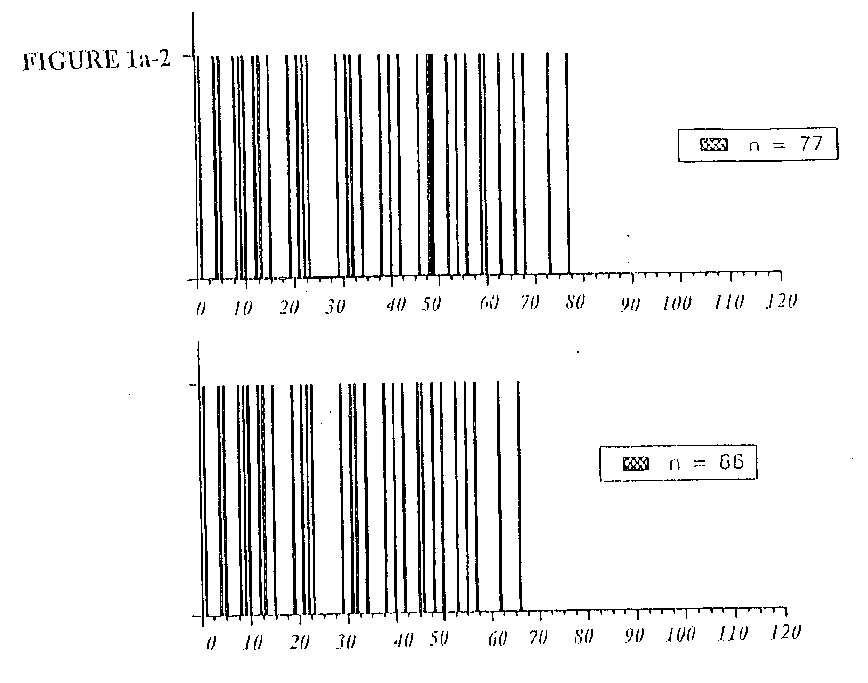

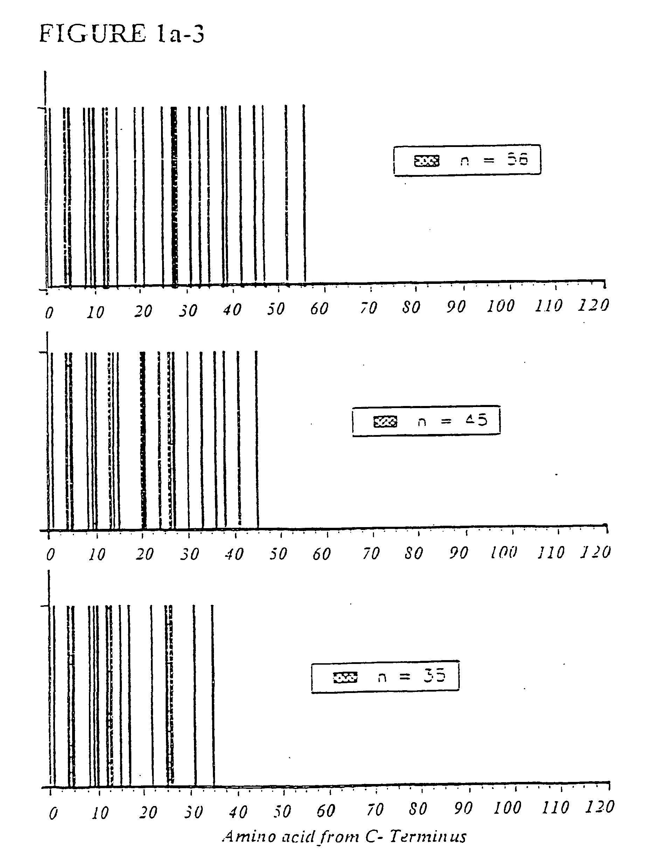

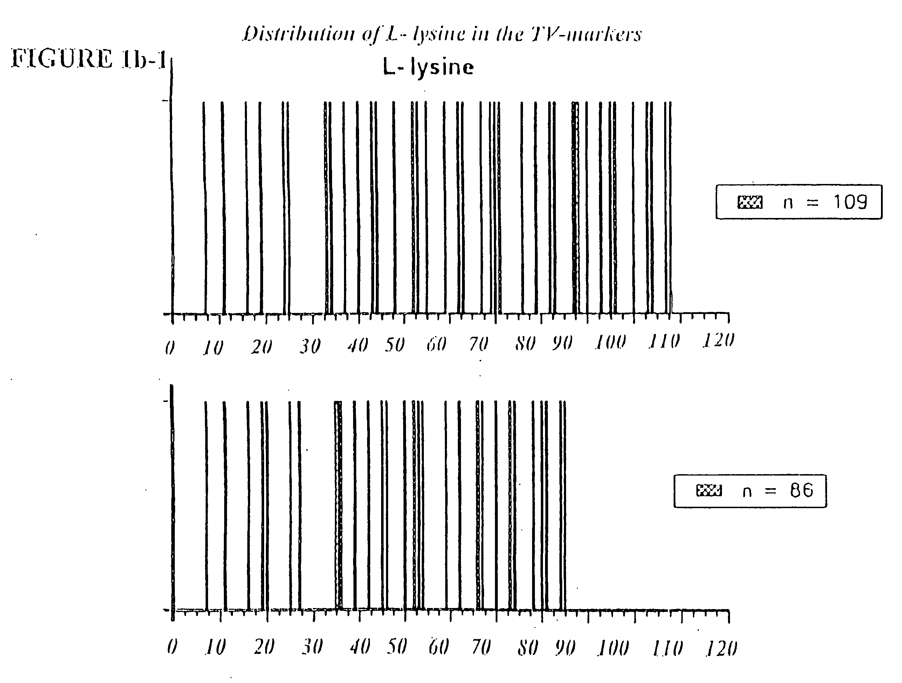

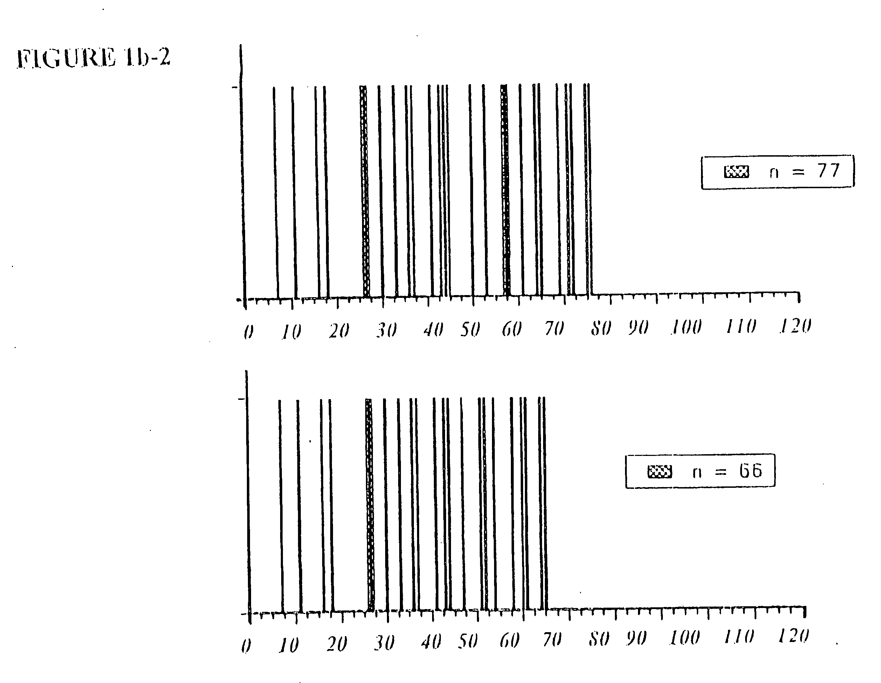

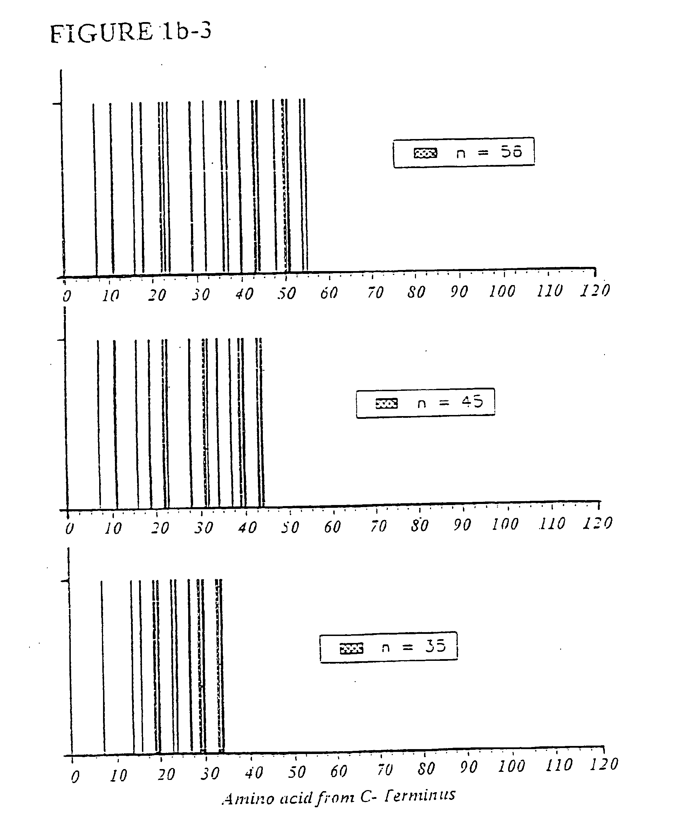

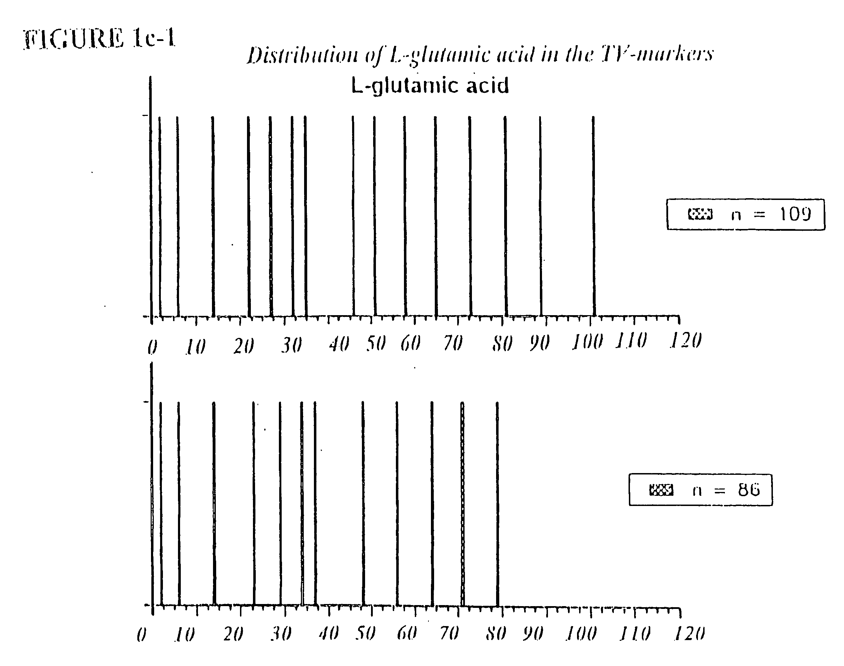

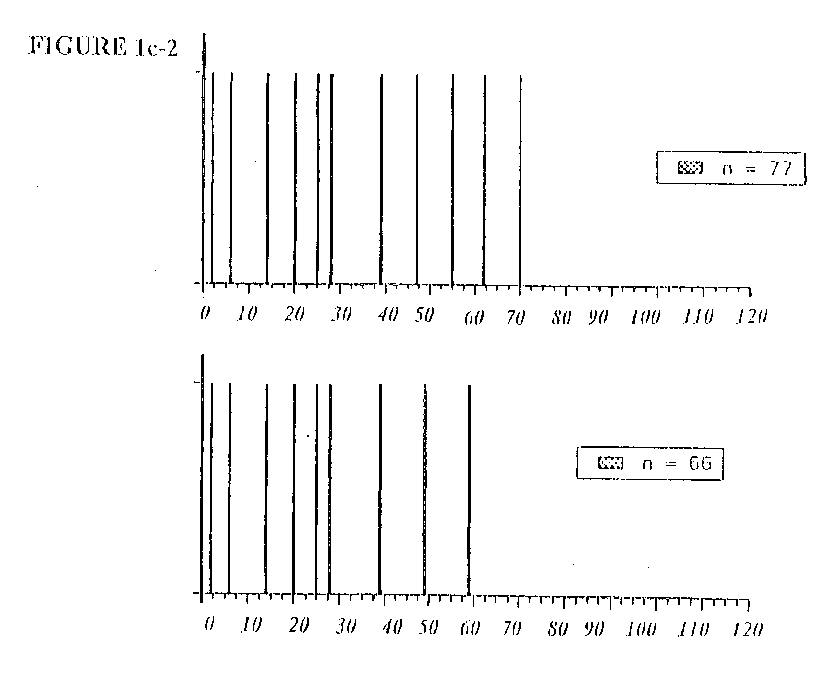

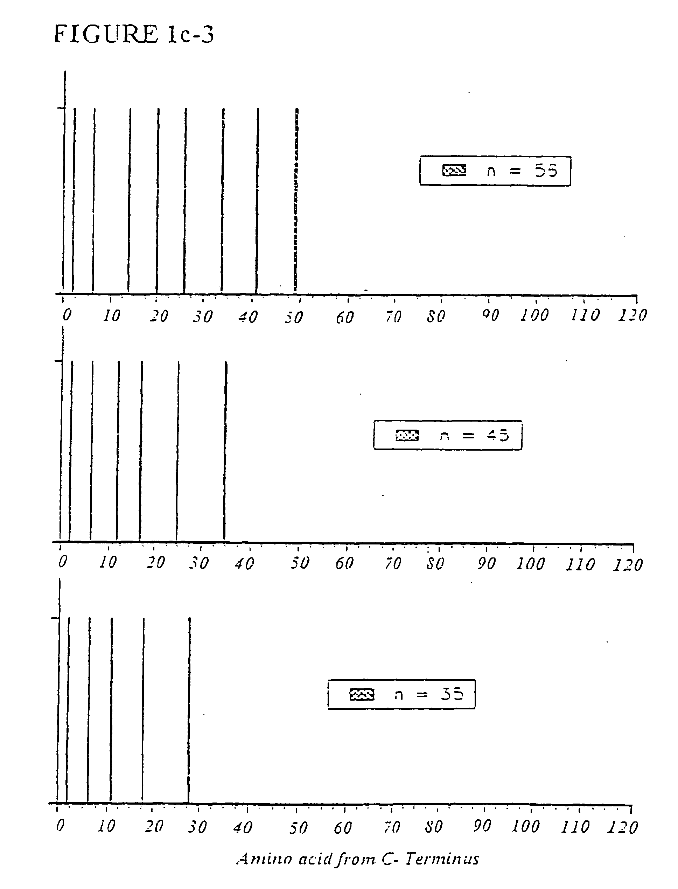

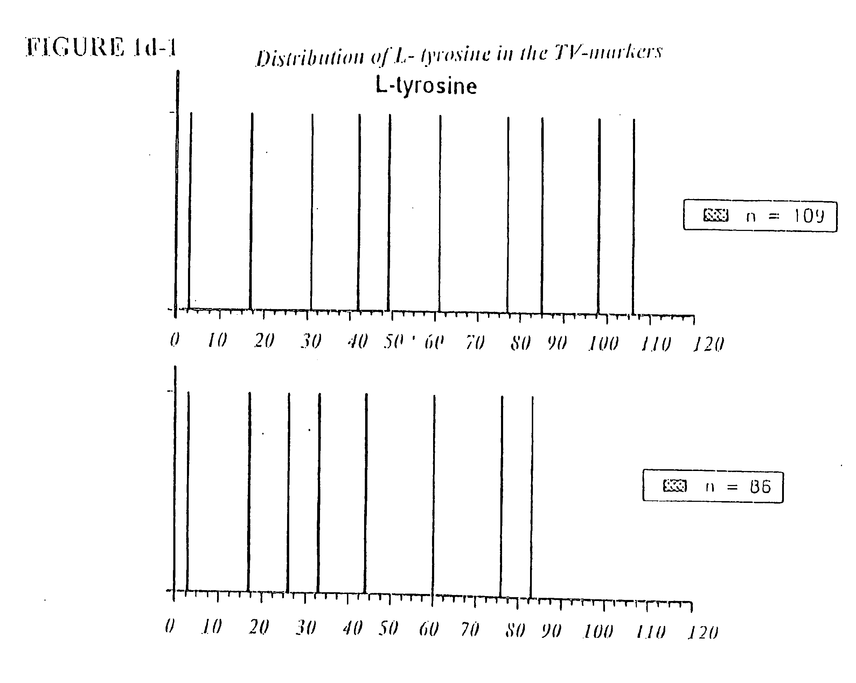

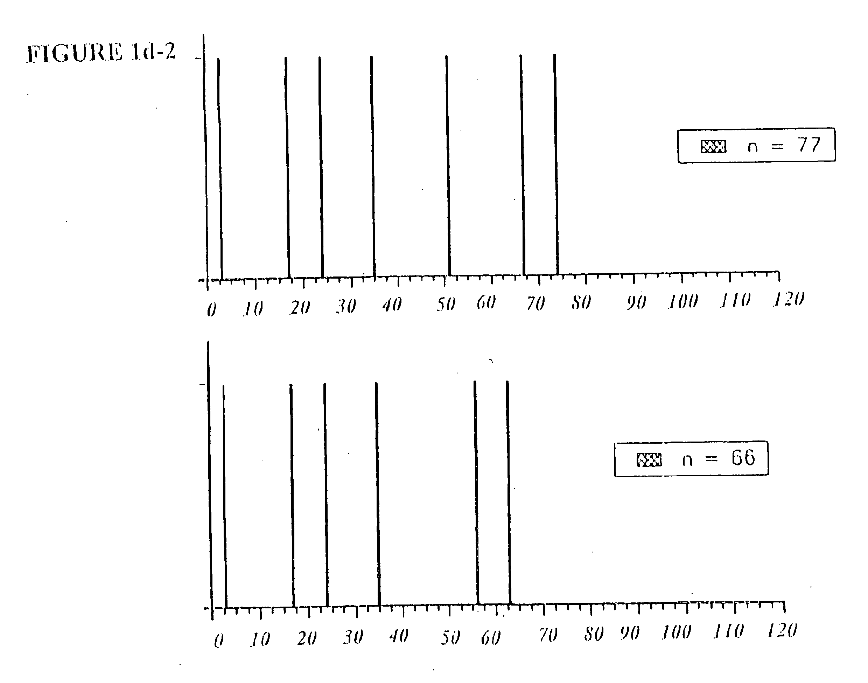

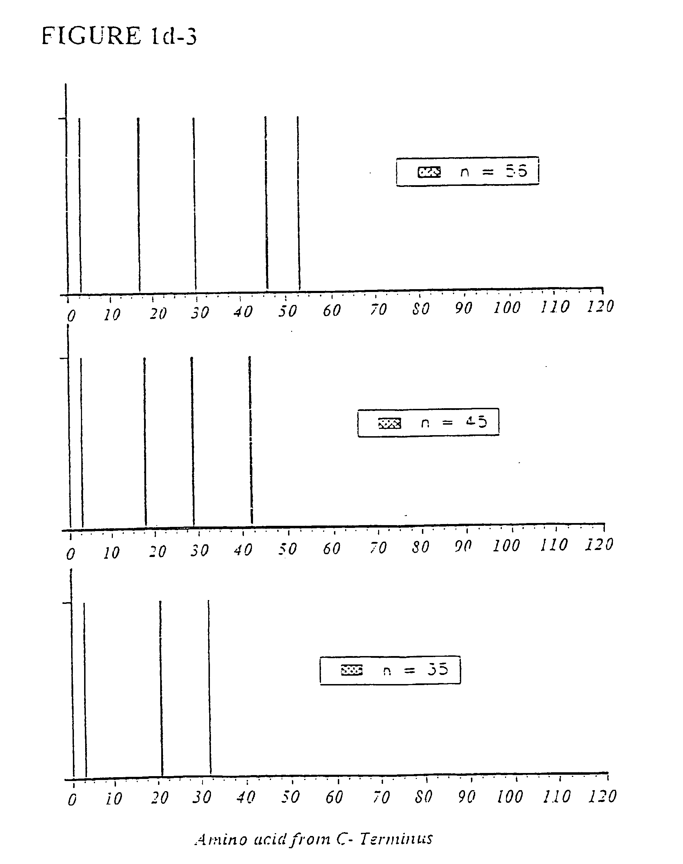

Figures 1a, 1b, 1 c and 1d provide the distribution of alanine, lysine, glutamic acid and tyrosine, respectively, in the TV-markers described in Table 2. The amino acid position is defined by the X-axis, with the first amino acid corresponding to the C-terminal position. The presence of an amino acid is indicated by a vertical bar at the indicated amino acid position.

**Confirmation of mass and sequence**

Mass Spectroscopy - Polypeptide samples were analyzed immediately after their synthesis using a VG platform mass spectrophotometer equipped with an electronspray ion source. Several months later the analysis was repeated at TEVA using a PE-Sciex AP1300 mass spectrophotometer equipped with an electronspray ion source (Table 3, first preparation). These results indicate that each polypeptide TV-marker has a single, main component with the intended molecular mass.

**Table 3. Mass Spectroscopy of Sequence-Defined Polypeptides**

| Polypeptide | Designed molecular mass (daltons) | Determined molecular mass - first preparation (daltons) | Determined molecular mass - second preparation (daltons) |
|---|---|---|---|
| TV-35 | 3757 | 3757 | 3757 |
| TV-45 | 4790 | 4790 | 4790 |
| TV-56 | 6008 | 6008 | 6008 |
| TV-66 | 7040 | 7041 | 7040 |
| TV-77 | 8259 | 8259 | 8259 |
| TV-86 | 9220 | 9220 | 9220 |

(continued)

| Polypeptide | Designed molecular mass (daltons) | Determined molecular mass - first preparation (daltons) | Determined molecular mass - second preparation (daltons) |
|---|---|---|---|
| TV-109" | 11727 | 11728 | 11727 |

\* The 109-mer was further purified by fractionation on a reversed-phase column. Three fractions were collected and fraction number 2 was designated for calibration purposes and referred to as TV-109.

A second batch of markers was prepared. Mass spectroscopy confirmed that the polypeptides of the second preparation were identical to the polypeptides of the first preparation (Table 3, second preparation). The similarity between the two preparations was also confirmed by chromatography on Superose 12. Each of the markers eluted with a sharp peak at a distinct retention time, regardless of the batch analyzed. Hence, the TV-markers of the present invention can be synthesized with reproducible mass.

Edman degradation - The intended sequence of the polypeptides was confirmed by Edman degradation analysis of the first preparation.

**Characterization of the polypeptides**

**Circular dichroism** - Structural similarity between the molecular weight markers and glatiramer acetate is a pre-requisite for an appropriate calibration of a molecular sizing column. Differences in polypeptide structure may result in different hydrodynamic size and consequently in altered retention time in the chromatographic system. The ellipticity, determined by circular dichroism, serves as a measure of the secondary structure of a polypeptide. When the ellipticity of the molecular weight markers and glatiramer acetate is similar, the structures of the two will be similar.

The molar ellipticity of the polypeptides was determined on a Jobin-Yvon CD spectrophotometer. Figure 2 and Table 4 show that the extent of molar ellipticity correlated with the molecular weight of the polypeptide. The shortest peptide exhibited the lowest ellipticity value. The molar ellipticity of the new markers was of the same order of magnitude as those of the currently used glatiramer acetate molecular weight markers. Note that while the exact molecular weight for the TV-markers was plotted, the averace-by-number molecular weight for the glatiramer acetate was used in the plot.

Thus, the new markers and glatiramer acetate possess similar structures and are therefore suitable for use as molecular weight markers for new preparations of glatiramer acetate.

**Table 4. Molecular Ellipticity**

| MW marker | MW (daltons) | M-ellip. (210nm) |
|---|---|---|
| **TV-markers** | | |
| TV-35 | 3757 | -1.5367 |
| TV-45 | 4790 | -2.1651 |
| TV-56 | 6008 | -3.9658 |
| TV-66 | 7040 | -3.5172 |
| TV-77 | 8259 | -4.8365 |
| TV-86 | 9220 | -5.4546 |
| TV-109 | 11727 | -6.818 |
| **glatiramer acetate** | | |
| BD 743 | 3700 | -2.0186 |
| BD 714 | 5600 | -4.4182 |
| BD 681 | 6600 | -5.2019 |
| BD 577 | 7000 | -6.0153 |
| 56895 | 8000 | -6.9062 |
| 90995 | 8500 | -9.1736 |

(continued)

| glatiramer acetate | | |
|---|---|---|
| BD 655 | 8900 | -8.8576 |

These analytical data indicate that the synthesized TV-marker polypeptides exhibit a substantial degree of similarity to the currently used glatiramer acetate molecular weight markers. The amino acid content is within glatiramer acetate specifications. The new polypeptides and the glatiramer acetate molecular weight markers have similar secondary structure, expressed as molar ellipticity. Consequently, TV-markers are expected to migrate or elute in a gel permeation chromatographic (GPC) system, such as Superose 12, like a glatiramer acetate preparation.

**EXAMPLE 2**

**Superose 12 Column Calibration with TV-markers**

TV-markers and a glatiramer acetate preparation are expected to demonstrate a similar correlation between relative retention time (RRT) and log molecular weight. The TV-markers were chromatographed on several Superose 12 columns. The peak retention time for each of the polypeptides was recorded. The linear correlation between Log Molecular Weight (MW) and the Relative Retention Time (RRT) was calculated as follows: RRT = B1 + B2 x LogMW (see Fig. 3a and Table 5).

The recently introduced Millennium-based data acquisition system (Waters Corp., Milford, MA) provides integrated calibration of GPC columns. The algorithm for the calibration is based on the retention time and is given by the equation:

$$LogMW = A + B \times RT \quad or \quad MW = 10^{(A + B \times RT)}$$

where MW is the molecular weight, RT is the retention time, A and B, respectively, are the intercept and the slope of the calculated regression function (Fig. 3b, Table 5).

The results obtained by this algorithm are practically identical to those obtained with the currently applied algorithm, based on RRT. In the effort to automate procedures, the Millennium-based data acquisition system was employed to perform the calibration using the TV-markers. The analytical methods were updated accordingly.

A good correlation ($r^2 > 0.98$) was obtained between log MW and RRT, although the points do not distribute evenly around the regression line. This distribution is due to the differences in the ellipticity of the various markers, as is also observed for the glatiramer acetate. The somewhat deviant-from-linearity low molecular weight marker cannot be excluded because the regression must cover values down to 2500 daltons for the first standard deviation (+ 1 SD) distribution parameter. This is a general trait of all shorter peptides - they are less helical and more linear.

For the calibration based on the glatiramer acetate molecular weight markers, the intercept (B1) and slope (B2) were, respectively, 1.7415 and -0.2784. This compares favorably with the calibration values obtained with TV-markers (B1 = 1.6996; B2 = -0.2705). The molecular weights obtained using the two calibration sets within the specification range differed by, typically, not more than 20% in the low molecular weight range and by not more than 12% in the RRT specification range of the peak (average molecular weight). This relatively small difference supports the claim that these markers can replace the currently used glatiramer acetate molecular weight markers without significant change in the reported molecular weight values.

**Table 5a. Calibration by glatiramer acetate MW-markers**

| Marker | MW | LOG MW | PEAK RT | RRT* |
|---|---|---|---|---|
| TV-35 | 3757 | 3.575 | 28.97 | 0.728 |
| TV-45 | 4790 | 3.68 | 27.96 | 0.703 |
| TV-56 | 6008 | 3.779 | 27.12 | 0.682 |
| TV-66 | 7040 | 3.848 | 26.32 | 0.662 |
| TV-77 | 8259 | 3.917 | 25.56 | 0.643 |
| TV-86 | 9220 | 3.965 | 24.93 | 0.627 |

(continued)

| Marker | MW | LOG MW | PEAK RT | RRT* |
|---|---|---|---|---|
| TV-109 | 11727 | 4.069 | 23.57 | 0.593 |
| INTERCEPT | ** A | 6.2516 | *** B1 | 1.6996 |
| SLOPE | B | -0.0918 | B2 | -0.2705 |
| $r^2$ | | 0.9927 | | 0.9923 |

* RRT = RT / RTAcetone
** calculated according Millennium equation: log MW=A + B x RT
*** calculated according to equation: RRT = $B_1$ + $B_2$ x log MW

Calibration based on TV-markers was compared to calibration based on glatiramer acetate molecular weight markers (Table 5b). The two calibrations were compared by calculating molecular weight values for each calibration set in the RRT range of 0.5 to 0.8. The TV-marker calibration set included a fraction of TV-109 which was purified by reversed phase chromatography prior to use for column calibration.

**Table 5b. Column Calibration by TV-markers**

| RRT | RT* (min) | Glatir.Ac. (MW1) | TV(0.1) (MWm) | Difference (MWm-MW1) | |
|---|---|---|---|---|---|
| | | Daltons | Daltons | Daltons | % |
| 0.5 | 19.89 | 28800 | 26700 | -2100 | -7.3% |
| 0.51 | 20.28 | 26500 | 24500 | -2000 | -7.5% |
| 0.52 | 20.68 | 24400 | 22600 | -1800 | -7.4% |
| 0.53 | 21.08 | 22500 | 20700 | -1800 | -8.0% |
| 0.54 | 21.48 | 20700 | 19100 | -1000 | -7.7% |
| 0.55 | 21.87 | 19000 | 17500 | -1500 | -7.9% |
| 0.56 | 22.27 | 17500 | 16100 | -1400 | -8.0% |
| 0.57 | 22.67 | 16100 | 14800 | -1300 | -8.1% |
| 0.58 | 23.07 | 14900 | 13600 | -1300 | -8.7% |
| 0.59 | 23.46 | 13700 | 12500 | -1200 | -8.8% |
| 0.6 | 23.85 | 12600 | 11500 | -1100 | -8.7% |
| 0.61 | 24.26 | 11600 | 10600 | -1000 | -8.7% |
| 0.62 | 24.66 | 10700 | 9700 | -1000 | -9.3% |
| 0.63 | 25.06 | 9800 | 9000 | -800 | -8.2% |
| 0.64 | 25.45 | 9000 | 8200 | -800 | -8.9% |
| 0.65 | 25.85 | 8300 | 7600 | -700 | -8.4% |
| 0.66 | 26.25 | 7700 | 7000 | -700 | -9.1 |
| 0.57 | 25.65 | 7100 | 6400 | -700 | -9.9 |
| 0.58 | 27.04 | 6600 | 6900 | -600 | -9.2% |
| 0.69 | 27.44 | 6000 | 5400 | -600 | -10.0% |
| 0.70 | 27.84 | 5500 | 5000 | -500 | -9.1 % |
| 0.71 | 28.24 | 5100 | 4600 | -500 | -9.8% |
| 0.72 | 28-63 | 4700 | 4200 | -500 | -10.6% |
| 0.73 | 29.03 | 4300 | 3900 | -400 | -9.3% |

(continued)

| RRT | RT* (min) | Glatir.Ac. (MW1) | TV(0.1) (MWm) | Difference (MWm-MW1) | |
|---|---|---|---|---|---|
| | | Daltons | Daltons | Daltons | % |
| 0.74 | 29.43 | 4000 | 3600 | -400 | -10.0% |
| 0.75 | 29.63 | 3600 | 3300 | -300 | -8.3% |
| 0.76 | 30.23 | 3400 | 3000 | -400 | -11.8% |
| 0.77 | 30.62 | 3100 | 2800 | -300 | -9.7% |
| 0.78 | 31.02 | 2800 | 2500 | -300 | -10.7% |
| 0.79 | 31.42 | 2600 | 2300 | -300 | -11.5% |
| 0.80 | 31.82 | 2400 | 2100 | -300 | -12.5% |

Purity of TV markers - Three of the markers (TV-66, TV-77 and TV-86) were further purified by reversed phase chromatography. Three fractions were obtained for each marker. The middle fraction containing the major portion of the peak was chromatographed on the Superose 12 system in comparison to the unfractionated markers (Table 6). TV markers were size chromatographed without purification (Regular) and after purification by reversed-phase chromatography (Purified). Peak retention times were determined and the differences were calculated. The peak retention time remained unaffected by the degree of purity. Therefore, the final product of the synthesis is useful for accurate calibration and extra purification is not required.

**Table 6. Effect of Purification on Retention Time**

| TV-marker | Retention Time (RT) (min) | | Difference (%) |
|---|---|---|---|
| | Regular | Purified | |
| TV-66 | 26.200 | 26.233 | -0.13% |
| TV-77 | 25.450 | 25.450 | 0.00% |
| TV-86 | 24.867 | 24.850 | 0.07% |

Consistency in reported values (Cross-validation) - Six batches of glatiramer acetate, manufactured in 1993 and 1994. were reanalyzed by GPC calibrated with the TV-markers. Their average molecular weight and the molecular weight distribution was compared to the values reported at the time of their release. Table 7 shows a comparison of molecular weight data from the original certificate of analysis and molecular weight data obtained using a Supercse 12 column calibrated with TV-markers. The differences in reported values are typically less than 10%.

**Table 7. Comparison of Molecular Weight Determinations**

| Cop 1 preparation | | MW Millennium | MW CoA | % difference |
|---|---|---|---|---|
| | | | | |
| | average | 10250 | 9900 | -3.5% |
| 00193 | - 1 SD | 20950 | 19100 | -9.7% |
| | + 1 SD | 51000 | 4800 | -6.3% |
| | | | | |
| | average | 6700 | 6550 | -2.3% |
| 00594 | -1 SD | 15700 | 15100 | -4.0% |
| | +1 SD | 3600 | 3400 | -5.9% |
| | | | | |

(continued)

| Cop 1 preparation | | MW Millennium | MW CoA | % difference |
|---|---|---|---|---|
| 00993 | average | 9200 | 8600 | -7.0% |
| | -1 SD | 18500 | 17350 | -6.9% |
| | +1 SD | 4700 | 4400 | -6.8% |
| | | | | |
| 04194 | average | 6100 | 6150 | 0.8% |
| | -1 SD | 12600 | 12500 | -0.8% |
| | +1 SD | 3200 | 3200 | 0.0% |
| | | | | |
| 01793 | average | 8800 | 3300 | -6.0% |
| | -1 SD | 18100 | 17300 | -4.6% |
| | +1 SD | 5200 | 4750 | -9.5% |
| | | | | |
| 05494 | average | 8100 | 8300 | 2.4% |
| | -1 SD | 17800 | 17450 | -2.0% |
| | +1 SD | 4100 | 4100 | 0.0% |

Stability of markers in solution - TV-markers were chromatographed four times over a period of 24 hours. All markers were kept as solutions at room temperature and were analyzed at 8 hour intervals. Table 8 shows the peak retention time measured for the TV-markers at each of the four time points. At a concentration of 0.1 mg/ml, the TV-markers were stable in solution for at least 24 hours at room temperature.

**Table 8. Stability of TV-markers in solution at room temperature.**

| | Peak Retention Time (min) | | | | Average | RSD |
|---|---|---|---|---|---|---|
| TV-35 | 29.883 | 29.883 | 29.900 | 29.950 | 29.904 | 0.106% |
| TV-45 | 28.933 | 28.917 | 28.917 | 28.933 | 28.925 | 0.032% |
| TV-56 | 28.250 | 28.217 | 28.283 | 28.250 | 28.250 | 0.095% |
| TV-66 | 27.400 | 27.350 | 27.433 | 27.433 | 27.404 | 0.143% |
| TV-77 | 26.750 | 26.700 | 26.750 | 26.783 | 26.746 | 0.128% |
| TV-86 | 26.117 | 26.100 | 26.150 | 26.150 | 26.129 | 0.095% |
| TV-109Fr-11 | 24.783 | 24.850 | 24.883 | 24.850 | 24.842 | 0.169% |

In addition, solutions of the markers were stored for up to 3 ½ months under various storage conditions (2-8 °C, -10 to -20°C, with/without azide). TV-markers are stable for at least 3 months when stored as frozen solutions (Table 9). As a precaution it was decided to allow storage of frozen solutions for two months.

Lyophilized TV-markers are stable for at least two years according to accumulated stability data.

**Table 9. Stability of TV-markers at-10° to -20°C.**

| Date of calibration: | | 22-May-97 | 09-Jul-97 | 04-step-97 |
|---|---|---|---|---|
| Interval (days) | | - | **48** | 105 |
| **Marker** | **MW** | RT | RT | RT |
| TV-35 | 3757 | 28.857 | 28.867 | 28.957 |
| TV-45 | 4790 | 27.833 | 27.917 | 27.950 |

(continued)

| Date of calibration: | | 22-May-97 | 09-Jul-97 | 04-step-97 |
|---|---|---|---|---|
| Interval (days) | | - | **48** | 105 |
| **Marker** | **MW** | RT | RT | RT |
| TV-56 | 6008 | 27.076 | 27.133 | 27.100 |
| TV-66 | 7040 | 26.233 | 26.317 | 26.300 |
| TV-77 | 8259 | 25.467 | 25.617 | 25.550 |
| TV-86 | 9220 | 24.883 | 25.017 | 24.950 |
| TV-109 | 11727 | 23.500 | 23.650 | 23.583 |

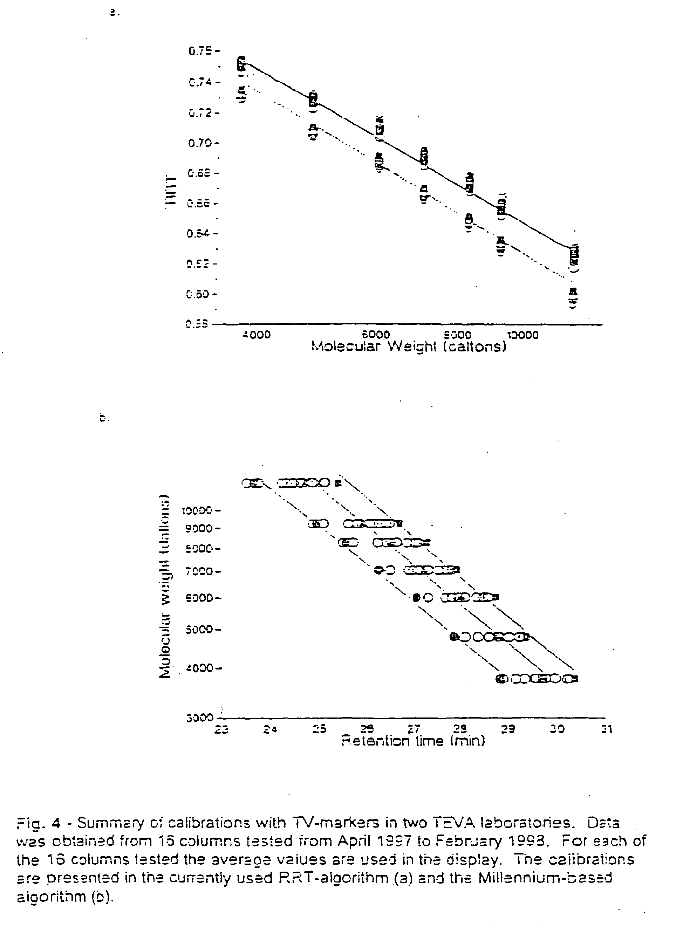

Summary of calibration data - Overall, the TV-markers were analyzed 53 times in two laboratories. A summary of the data is presented in Fig. 4 and Table 10. The differences observed among the individual runs (Fig. 4) reflect variations between columns rather than differences between the participating laboratories. This is indicated in Fig. 4 by the use of different symbols for some of the runs. Calibration constants in Table 10 were calcualted using the Millennium equation for data obtained for 53 calibration sets injected into 16 columns,

**Table 10. Calibration constants obtained in Plantex and Abic Labs**

| | | RT | | | RT | | | |
|---|---|---|---|---|---|---|---|---|
| **Marker** | **MW** | **Mean** | **SD** | **RSD%** | **Min** | **Max** | **Mean-SD** | **Mean+ SD** |
| TV-35 | 3757 | 29.69 | 0.463 | 1.6% | 28.85 | 30.35 | 28.30 | 31.08 |
| TV-45 | 4790 | 28.72 | 0.481 | 1.7% | 27.88 | 29.40 | 27.28 | 30.16 |
| TV-56 | 6008 | 27.99 | 0.520 | 1.9% | 27.08 | 28.77 | 26.43 | 29.55 |
| TV-66 | 7040 | 27.19 | 0.525 | 1.9% | 26.26 | 27.96 | 25.61 | 23.77 |
| TV-77 | 8259 | 26.49 | 0.550 | 2.1% | 25.51 | 27.33 | 24.84 | 28.14 |
| TV-86 | 9220 | 25.89 | 0.556 | 2.1% | 24.89 | 26.72 | 24.22 | 27.56 |
| TV-109 | 11727 | 24.56 | 0.557 | 2.3% | 23.53 | 25.41 | 22.89 | 26.23 |
| Intercept (A) | | 6.4706 | 0.12-20 | 1.9% | 6.2561 | 6.6500 | 6.1046 | 6.8366 |
| Slope (B) | | -0.0969 | 0.0032 | -3.3% | -0.1014 | -0.0919 | -0.1064 | -0.0873 |
| $r^2$ | | 0.9901 | 0.0022 | 0.2% | 0.9868 | 0.9328 | 0.9836 | 0.9967 |

**Molecular weight distribution of a glatiramer acetate preparation-**Molecular weight was determined for a batch of glatiramer acetate (BN 90995). Table 11 summarizes data obtained from 16 determinations on TV-marker-calibrated columns.

**Table11a. RT of glatiramer acetate (BN 90995)**

| | Average | SD | RSD% |
|---|---|---|---|
| Peak | 26.208 | 0.434 | 1.66 |
| -2SD (2.5%) | 19.865 | 0.528 | 2.66 |
| -1SD (16%) | 22.578 | 0.477 | 2.11 |
| +1SD (84%) | 28.934 | 0.324 | 1.12 |

**Table 11b. RRT of glatiramer acetate (BN 90995)**

|  | Average | SD | RSD% |
|---|---|---|---|
| Peak | 0.664 | 0.014 | 2.09 |
| -2SD (2.5%) | 0.503 | 0.016 | 3.09 |
| -1SD (16%) | 0.572 | 0.015 | 2.54 |
| +1SD (84%) | 0.733 | 0.011 | 1.53 |

**Table 11 c. MW (Daltons) of glatiramer acetate (BN 90995)**

| Date | Average | SD | RSD% |
|---|---|---|---|
| Peak | 7459 | 146 | 1.95 |
| -1SD (16%) | 16622 | 466 | 2.80 |
| +1SD (84%) | 4089 | 77 | 1.89 |

Tne application of a molecular weight and sequence-defined set of markers for the calibration of the Superose 12 column has several advantages over the currently used glatiramer acetate molecular weight markers.

First, the use of solid phase synthesis assures consistency among the various preparations of each batch. Mass spectroscopy results (Table 3) confirmed the reproducibility of the synthesis. This consistency provides improved accuracy in molecular weight determinations.

Second, the current calibration is based on the determination of the RRT at 50% of the peak area for each of the glatiramer acetate molecular weight markers. The new markers elute as sharp peaks. Their use in calibration is more accurate than the calculated retention time at 50% of the area of a broad peak.

Third, the use of markers having molecular weights defined by predetermined sequence precludes any uncertainty which might accompany the use of markers whose molecular weight is determined by inexact measurement of physical properties.

Fourth, the calibration procedure facilitates normalization of columns for molecular weight determinations, regardless of minor changes between columns lots, age or instrumentation.

## Claims

1. A process for preparing a pharmaceutical product comprising glatiramer acetate having an average molecular weight from 4,000 to 13,000 Daltons which process comprises the steps of

   obtaining a glatiramer acetate preparation;
   determining the average molecular weight of the glatiramer acetate preparation using a chromatographic apparatus which is calibrated using a plurality of molecular weight markers to establish a linear relationship between the retention time of the molecular weight markers on the chromatographic apparatus and the log of the molecular weight of the molecular weight markers, wherein each of the molecular weight markers is a polypeptide consisting of alanine, glutamic acid, tyrosine and lysine and having a predetermined amino acid sequence and defined molecular weight; and
   using the glatiramer acetate preparation to prepare the pharmaceutical product if the glatiramer acetate preparation has an average molecular weight from 4,000 to 13,000 Daltons.

2. A process for certifying glatiramer acetate for use in a pharmaceutical product comprising glatiramer acetate having an average molecular weight from 4,000 to 13,000 Daltons which process comprises the steps of

   determining the average molecular weight of the glatiramer acetate using a chromatographic apparatus which is calibrated using a plurality of molecular weight markers to establish a linear relationship between the retention time of the molecular weight markers on the chromatographic apparatus and the log of the molecular weight of the molecular weight markers, wherein each of the molecular weight markers is a polypeptide consisting of alanine, glutamic acid, tyrosine and lysine and having a predetermined amino acid sequence and defined molecular weight; and
   certifying the glatiramer acetate for use in the pharmaceutical product if the glatiramer acetate has an average molecular weight from 4,000 to 13,000 Daltons.

3. The process of claim I or 2, wherein in the molecular weight markers the molar fraction of alanine is 0.38 to 0.5, of glutamic acid is 0.13 to 0.15, of tyrosine is 0.08 to 0.10 and of lysine is 0.3 to 0.4.

4. The process of any one of claims 1-3, wherein in the molecular weight markers the molar fraction of alanine is 0.422 to 0.444, of glutamic acid is 0.133 to 0.143, of tyrosine is 0.086 to 0.093 and of lysine is 0.333 to 0.349.

5. The process of any one of claims 1-4, wherein the chromatographic apparatus is a gel permeation chromatography column.

6. The process of any one of claims 1-4, wherein the chromatographic apparatus is a TSK column, a Sephadex column, a Sepharose column, or a Superose column.

7. The process of any one of claims 1-6, wherein one of the molecular weight markers is:

AKKYAKKEKAAKKAYKKEAKAKAAEAAAKEAAYEA (SEQ ID NO: *1);*
AKKYAKKAKAEKAKKAYAAEAKKAAKYEKAAAEKAAAKEAAYEA (SEQ ID NO: 2);

AKKYAKKEKAYAKKAEKAAKKAEAKAYKAAEAKKKAEAKYKAEAAKAAA KEAAYEA (SEQ ID NO: 3);

AKKYAKKEKAYAKAKKAEAKAAKKAKAEAKKYAKAAKAEKKEYAAAEAK YKAEAAKAAAKEAAYEA (SEQ ID NO: 4);

AKKYAKKEKAYAKKAEKAAKKAEAKAYKAAEAKKKAKAEAKKYAKAAKA EKKEYAAAEAKYKAEAAKAAAKEAAYEA (SEQ ID NO: 5);

AKKYAKKEKAYAKKAEKAAKKAEAKAYKAAEAKKKAKAEAKKYAKAAKA EKKEYAAAEAKYKAEAAKKAYKAEAAKAAAKEAAYEA (SEQ ID NO: 6); or

AKKYAKKAEKAYAKKAKAAKEKKAYAKKEAKAYKAAEAKKKAKAEAKKY AKEAAKAKKEAYKAEAKKYAKAAKAEKKEYAAAEAKKAEAAKAYKAEAAK A AAKEAAYEA (SEQ ID NO: 7),

wherein A represents alanine, K represents lysine, Y represents tyrosine, and E represents glutamic acid.

**8.** The process of any one of claims 1-6, wherein the plurality of molecular weight markers is:

AKKYAKKEKAAKKAYKKEAKAKAAEAAAKEAAYEA (SEQ ID NO: 1);
AKKYAKKAKAEKAKKAYKAAEAKKAAKYEKAAAEKAAAKEAAYEA (SEQ ID NO: 2);

AKKYAKKEKAYAKKAEKAAKKAEAKAYKAAEAKKKAEAKYKAEAAKAAA KEAAYEA (SEQ ID NO: 3);

AKKYAKKEKAYAKAKKAEAKAAKKAKAEAKKYAKAAKAEKKEYAAAEAK YKAEAAKAAAKEAAYEA (SEQ ID NO: 4);

AKKYAKKEKAYAKKAEKAAKKAEAKAYKAAEAKKKAKAEAKKYAKAAKA EKKEYAAAEAKYKAEAAKAAAKEAAYEA (SEQ ID NO: 5);

AKKYAKKEKAYAKKAEKAAKKAEAKAYKAAEAKKKAKAEAKKYAKAAKA EKKEYAAAEAKYKAEAAKKAYKAEAAKAAAKEAAYEA (SEQ ID NO: 6); and

AKKYAKKAEKAYAKKAKAAKEKKAYAKKEAKAYKAAEAKKKAKAEAKKY
AKEAAKAKKEAYKAEAKKYAKAAKAEKKEYAAAEAKKAEAAKAYKAEAAK
A AAKEAAYEA (SEQ ID NO: 7),

wherein A represents alanine, K represents lysine, Y represents tyrosine, and E represents glutamic acid.

**Patentansprüche**

1.  Verfahren zur Herstellung eines Arzneimittels, das Glatirameracetat mit einem mittleren Molekulargewicht von 4.000 bis 13.000 Dalton umfasst, wobei das Verfahren die folgenden Schritte umfasst:

    Erhalten einer Glatirameracetatformulierung;
    Bestimmen des mittleren Molekulargewichts der Glatirameracetatformulierung unter Verwendung eines chromatographischen Geräts, das mit Hilfe einer Vielzahl von Molekulargewichtsmarkern kalibriert wird, um einen linearen Zusammenhang zwischen der Retentionszeit der Molekulargewichtsmarker auf dem chromatographischen Gerät und dem Logarithmus des Molekulargewichts der Molekulargewichtsmarker herzustellen, wobei jeder der Molekulargewichtsmarker ein Polypeptid ist, das aus Alanin, Glutaminsäure, Tyrosin und Lysin besteht und eine vorgegebene Aminosäuresequenz und ein definiertes Molekulargewicht hat; und
    Verwenden der Glatirameracetatformulierung zur Herstellung des Arzneimittels, wenn die Glatirameracetatformulierung ein mittleres Molekulargewicht von 4.000 bis 13.000 Dalton hat.

2.  Verfahren zum Zertifizieren von Glatirameracetat für die Verwendung in einem Arzneimittel, das Glatirameracetat mit einem mittleren Molekulargewicht von 4.000 bis 13.000 Dalton umfasst, wobei das Verfahren die folgenden Schritte umfasst:

    Bestimmen des mittleren Molekulargwichts des Glatirameracetats unter Verwendung eines chromatographischen Geräts, das mit Hilfe einer Vielzahl von Molekulargewichtsmarkern kalibriert wird, um einen linearen Zusammenhang zwischen der Retentionszeit der Molekulargewichtsmarker auf dem chromatographischen Gerät und dem Logarithmus des Molekulargewichts der Molekulargewichtsmarker herzustellen, wobei jeder der Molekulargewichtsmarker ein Polypeptid ist, das aus Alanin, Glutaminsäure, Tyrosin und Lysin besteht und eine vorgegebene Aminosäuresequenz und ein definiertes Molekulargewicht hat; und
    Zertifizieren des Glatirameracetats für die Verwendung in dem Arzneimittel, wenn das Glatirameracetat ein mittleres Molekulargewicht von 4.000 bis 13.000 Dalton hat.

3.  Verfahren nach Anspruch 1 oder 2, wobei in den Molekulargewichtsmarkern der molare Anteil von Alanin 0,38 bis 0,5, der von Glutaminsäure 0,13 bis 0,15, der von Tyrosin 0,08 bis 0,10 und der von Lysin 0,3 bis 0,4 beträgt.

4.  Verfahren nach einem der Ansprüche 1 bis 3, wobei in den Molekulargewichtsmarkern der molare Anteil von Alanin 0,422 bis 0,444, der von Glutaminsäure 0,133 bis 0,143, der von Tyrosin 0,086 bis 0,093 und der von Lysin 0,333 bis 0,349 beträgt.

5.  Verfahren nach einem der Ansprüche 1 bis 4, wobei das chromatographische Gerät eine Gelpermeationschromatographie-Säule ist.

6.  Verfahren nach einem der Ansprüche 1 bis 4, wobei das chromatographische Gerät eine TSK-Säule, eine Sephadex-Säule, eine Sepharose-Säule oder eine Superose-Säule ist.

7.  Verfahren nach einem der Ansprüche 1 bis 6, wobei einer der Molekulargewichtsmarker
    AKKYAKKEKAAKKAYKKEAKAKAAEAAAKEAAYEA (SEQ ID NO: 1):
    AKKYAKKAKAEKAKKAYKAAEAKKAAKYEKAAAEKAAAKEAAYEA (SEQ ID NO: 2);

    AKKYAKKEKAYAKKAEKAAKKAEAKAYKAAEAKKKAEAKYKAEAAKAAA
    KEAAYEA (SEQ ID NO: 3);

AKKYAKKEKAYAKAKKAEAKAAKKAKAEAKKYAKAAKAEKKEYAAAEAK YKAEAAKAAAKEAAYEA (SEQ ID NO: 4);

AKKYAKKEKAYAKKAEKAAKKAEAKAYKAAEAKKKAKAEAKKYAKAAKA EKKEYAAAEAKYKAEAAKAAAKEAAYEA (SEQ ID NO: 5);

AKKYAKKEKAYAKKAEKAAKKAEAKAYKAAEAKKKAKAEAKKYAKAAKA EKKEYAAAEAKYKAEAAKKAYKAEAAKAAAKEAAYEA (SEQ ID NO: 6); oder

AKKYAKKAEKAYAKKAKAAKEKKAYAKKEAKAYKAAEAKKKAKAEAKKY AKEAAKAKKEAYKAEAKKYAKAAKAEKKEYAAAEAKKAEAAKAYKAEAAK A AAKEAAYEA (SEQ ID NO: 7)

ist,
wobei A Alanin ist, K Lysin ist, Y Tyrosin ist und E Glutaminsäure ist.

8. Verfahren nach einem der Ansprüche 1 bis 6, wobei die Vielzahl der Molekulargewichtsmarker
AKKYAKKEKAAKKAYKKEAKAKAAEAAAKEAAYEA (SEQ ID NO: 1);
AKKYAKKAKAEKAKKAYKAAEAKKAAKYEKAAAEKAAAKEAAYEA (SEQ ID NO: 2);

AKKYAKKEKAYAKKAEKAAKKAEAKAYKAAEAKKKAEAKYKAEAAKAAA KEAAYEA (SEQ ID NO: 3);

AKKYAKKEKAYAKAKKAEAKAAKKAKAEAKKYAKAAKAEKKEYAAAEAK YKAEAAKAAAKEAAYEA (SEQ ID NO: 4);

AKKYAKKEKAYAKKAEKAAKKAEAKAYKAAEAKKKAKAEAKKYAKAAKA EKKEYAAAEAKYKAEAAKAAAKEAAYEA (SEQ ID NO: 5);

AKKYAKKEKAYAKKAEKAAKKAEAKAYKAAEAKKKAKAEAKKYAKAAKA EKKEYAAAEAKYKAEAAKKAYKAEAAKAAAKEAAYEA (SEQ ID NO: 6); und

AKKYAKKAEKAYAKKAKAAKEKKAYAKKEAKAYKAAEAKKKAKAEAKKY AKEAAKAKKEAYKAEAKKYAKAAKAEKKEYAAAEAKKAEAAKAYKAEAAK A AAKEAAYEA (SEQ ID NO: 7)

ist,
wobei A Alanin ist, K Lysin ist, Y Tyrosin ist und E Glutaminsäure ist.

**Revendications**

1. Procédé destiné à préparer un produit pharmaceutique comprenant de l'acétate de glatiramère possédant un poids moléculaire moyen de 4 000 à 13 000 Daltons, procédé qui comprend les étapes consistant à :

   obtenir une préparation d'acétate de glatiramère ;
   déterminer le poids moléculaire moyen de la préparation d'acétate de glatiramère en utilisant un appareil de chromatographie qui est étalonné en utilisant une pluralité de marqueurs de poids moléculaire pour établir une relation linéaire entre le temps de séjour des marqueurs de poids moléculaire sur l'appareil de chromatographie et le logarithme du poids moléculaire des marqueurs de poids moléculaire, où chacun des marqueurs de poids moléculaire est un polypeptide constitué de l'alanine, de l'acide glutamique, de la tyrosine et de la lysine et possédant une séquence d'acides aminés prédéterminée et un poids moléculaire défini ; et
   utiliser la préparation d'acétate de glatiramère pour préparer le produit pharmaceutique si la préparation d'acétate de glatiramère possède un poids moléculaire moyen de 4 000 à 13 000 Daltons.

2. Procédé destiné à authentifier de l'acétate de glatiramère pour une utilisation dans un produit pharmaceutique comprenant de l'acétate de glatiramère possédant un poids moléculaire moyen de 4 000 à 13 000 Daltons, procédé qui comprend les étapes consistant à :

   déterminer le poids moléculaire moyen de l'acétate de glatiramère en utilisant un appareil de chromatographie qui est étalonné en utilisant une pluralité de marqueurs de poids moléculaire pour établir une relation linéaire entre le temps de séjour des marqueurs de poids moléculaire sur l'appareil de chromatographie et le logarithme du poids moléculaire des marqueurs de poids moléculaire, où chacun des marqueurs de poids moléculaire est un polypeptide constitué de l'alanine, de l'acide glutamique, de la tyrosine et de la lysine et possédant une séquence d'acides aminés prédéterminée et un poids moléculaire défini ; et
   authentifier l'acétate de glatiramère pour une utilisation dans le produit pharmaceutique si l'acétate de glatiramère possède un poids moléculaire moyen de 4 000 à 13 000 Daltons.

3. Procédé selon la revendication 1 ou 2, où, dans les marqueurs de poids moléculaire, la fraction molaire de l'alanine est de 0,38 à 0,5, de l'acide glutamique est de 0,13 à 0,15, de la tyrosine est de 0,08 à 0,10 et de la lysine est de 0,3 à 0,4.

4. Procédé selon l'une quelconque des revendications 1 à 3, où, dans les marqueurs de poids moléculaire, la fraction molaire de l'alanine est de 0,422 à 0,444, de l'acide glutamique est de 0,133 à 0,143, de la tyrosine est de 0,086 à 0,093 et de la lysine est de 0,333 à 0,349.

5. Procédé selon l'une quelconque des revendications 1 à 4, dans lequel l'appareil de chromatographie est une colonne de chromatographie par perméation de gel.

6. Procédé selon l'une quelconque des revendications 1 à 4, dans lequel l'appareil de chromatographie est une colonne TSK, une colonne de Sephadex, une colonne de Sepharose, ou une colonne de Superose.

7. Procédé selon l'une quelconque des revendications 1 à 6, dans lequel l'un des marqueurs de poids moléculaire est :

   AKKYAKKEKAAKKAYKKEAKAKAAEAAAKEAAYEA (SEQ ID NO : 1);
   AKKYAKKAKAEKAKKAYKAAEAKKAAKYEKAAAEKAAAKEAAYEA (SEQ ID NO : 2) ;

   AKKYAKKEKAYAKKAEKAAKKAEAKAYKAAEAKKKAEAKYKAEAA
   KAAAKEAAYEA (SEQ ID NO : 3) ;

   AKKYAKKEKAYAKAKKAEAKAAKKAKAEAKKYAKAAKAEKKEYAA
   AEAKYKAEAAKAAAKEAAYEA (SEQ ID NO : 4) ;

AKKYAKKEKAYAKKAEKAAKKAEAKAYKAAEAKKKAKAEAKKYAK
AAKAEKKEYAAAEAKYKAEAAKAAAKEAAYEA (SEQ ID NO : 5) ;

AKKYAKKEKAYAKKAEKAAKKAEAKAYKAAEAKKKAKAEAKKYA
KAAKAEKKEYAAAEAKYKAEAAKKAYKAEAAKAAAKEAAYEA
(SEQ ID NO : 6 ) ; ou

AKKYAKKAEKAYAKKAKAAKEKKAYAKKEAKAYKAAEAKKKAKAE
AKKYAKEAAKAKKEAYKAEAKKYAKAAKAEKKEYAAAEAKKAEAAK
AYKAEAAKA AAKEAAYEA (SEQ ID NO : 7),

où A représente de l'alanine, K représente de la lysine, Y représente de la tyrosine, et E représente de l'acide glutamique.

8. Procédé selon l'une quelconque des revendications 1 à 6, dans lequel la pluralité de marqueurs de poids moléculaire est :

AKKYAKKEKAAKKAYKKEAKAKAAEAAAKEAAYEA (SEQ ID NO : 1) ;
AKKYAKKAKAEKAKKAYKAAEAKKAAKYEKAAAEKAAAKEAAYEA (SEQ ID NO : 2) ;

AKKYAKKEKAYAKKAEKAAKKAEAKAYKAAEAKKKAEAKYKAEAA
KAAAKEAAYEA (SEQ ID NO : 3) ;

AKKYAKKEKAYAKAKKAEAKAAKKAKAEAKKYAKAAKAEKKEYAA
AEAKYKAEAAKAAAKEAAYEA (SEQ ID NO : 4) ;

AKKYAKKEKAYAKKAEKAAKKAEAKAYKAAEAKKKAKAEAKKYAK
AAKAEKKEYAAAEAKYKAEAAKAAAKEAAYEA (SEQ ID NO : 5) ;

AKKYAKKEKAYAKKAEKAAKKAEAKAYKAAEAKKKAKAEAKKYAKAAKA
EKKEYAAAEAKYKAEAAKKAYKAEAAKAAAKEAAYEA (SEQ ID NO : 6 ) ; et

AKKYAKKAEKAYAKKAKAAKEKKAYAKKEAKAYKAAEAKKKAKAE
AKKYAKEAAKAKKEAYKAEAKKYAKAAKAEKKEYAAAEAKKAEAAK
AYKAEAAKA AAKEAAYEA (SEQ ID NO : 7),

où A représente de l'alanine, K représente de la lysine, Y représente de la tyrosine, et E représente de l'acide glutamique.

## Distribution of L-alanine in the TV-markers

### L-alanine

n = 109

0    10    20    30    40    50    60    70    80    90    100    110    120

n = 86

0    10    20    30    40    50    60    70    80    90    100    110    120

FIGURE 1a-2

n = 77

n = 66

# FIGURE 1a-3

Amino acid from C- Terminus

FIGURE 1b-1

Distribution of L-lysine in the TV-markers

L-lysine

n = 109

0  10  20  30  40  50  60  70  80  90  100  110  120

n = 86

0  10  20  30  40  50  60  70  80  90  100  110  120

FIGURE 1b-2

FIGURE 1b-3

Amino acid from C- Terminus

## *Distribution of L-glutamic acid in the TV-markers*
## L-glutamic acid

n = 109

n = 86

EP 2 239 269 B1

FIGURE 1c-2

FIGURE 1c-3

Amino acid from C- Terminus

## FIGURE 1d-1

*Distribution of L- tyrosine in the TV-markers*

**L-tyrosine**

n = 109

n = 86

FIGURE 1d-2

n = 77

0  10  20  30  40  50  60  70  80  90  100  110  120

n = 66

0  10  20  30  40  50  60  70  80  90  100  110  120

EP 2 239 269 B1

# FIGURE 1d-3

n = 55

n = 45

n = 35

*Amino acid from C- Terminus*

FIGURE 2

Figure 2 Ellipticity of the TV-markers and the currently used glatiramer acetate molecular weight markers as a function of their molecular weight. The experimental CD values are represented below.

a)

b)

Fig. 3 - Calibration of a Superose 12 column with the set of 7 TV markers displayed calculated as the RRT-based algorithm (a) and the Millennium-based algorithm (b).

a.

b.

Fig. 4 - Summary of calibrations with TV-markers in two TEVA laboratories. Data
was obtained from 16 columns tested from April 1997 to February 1998. For each of
the 16 columns tested the average values are used in the display. The calibrations
are presented in the currently used RRT-algorithm (a) and the Millennium-based
algorithm (b).

## Fig. 5

### ELISA of "mono-COP-1"s using inhibition-type immunoassay

Legend:
- —※— 03494
- —○— TV-35
- —◑— TV-56
- —⊟— TV-77
- —△— TV-86

Y-axis: Absorbance ratio (405 nm)

X-axis: Soluble hapten (μg/mL)

EP 2 239 269 B1

**EP 2 239 269 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5800808 A

- US 60101693 B

**Non-patent literature cited in the description**

- *J Med. Sci,* 1997, vol. 33, 280-286

SEQUENCE LISTING

<110> YEDA RESEARCH AND DEVELOPMENT CO., LTD.

<120> COPOLYMER 1 RELATED POLYPETIDES FOR USE AS MOLECULAR WEIGHT MARKERS AND FOR THERAPEUTIC USE

<130> P1594 **EP/2** S3

<140>
<141> 1999-09-24

<150> US 60/101,693
<151> 1998-09-25

<160> 7

<170> PatentIn version 3.0

<210> 1
<211> 35
<212> PRT
<213> ARTIFICIAL SEQUENCE

<220>
<221> Source
<222> (1)..(35)
<223> /note="Description of artificial sequence: synthetic Peptide"

<400> 1

```
Ala Lys Lys Tyr Ala Lys Lys Glu Lys Ala Ala Lys Lys Ala Tyr Lys
1               5               10              15

Lys Glu Ala Lys Ala Lys Ala Ala Glu Ala Ala Ala Lys Glu Ala Ala
            20              25              30

Tyr Glu Ala
        35
```

<210> 2
<211> 45
<212> PRT
<213> ARTIFICIAL SEQUENCE

<220>
<221> Source
<222> (1)..(45)
<223> /note="Description of artificial sequence: synthetic peptide"

<400> 2

```
Ala Lys Lys Tyr Ala Lys Lys Ala Lys Ala Glu Lys Ala Lys Lys Ala
1               5               10              15

Tyr Lys Ala Ala Glu Ala Lys Lys Ala Ala Lys Tyr Glu Lys Ala Ala
            20              25              30

Ala Glu Lys Ala Ala Ala Lys Glu Ala Ala Tyr Glu Ala
        35              40              45
```

<210> 3
<211> 56
<212> PRT
<213> ARTIFICIAL SEQUENCE

<220>
<221> Source
<222> (1)..(56)
<223> /note="Description of artificial sequence: synthetic septide"

<400> 3

```
Ala Lys Lys Tyr Ala Lys Lys Glu Lys Ala Tyr Ala Lys Lys Ala Glu
1               5               10              15

Lys Ala Ala Lys Lys Ala Glu Ala Lys Ala Tyr Lys Ala Ala Glu Ala
            20              25              30

Lys Lys Lys Ala Glu Ala Lys Tyr Lys Ala Glu Ala Ala Lys Ala Ala
        35              40              45

Ala Lys Glu Ala Ala Tyr Glu Ala
        50              55
```

<210> 4
<211> 66
<212> PRT
<213> ARTIFCIAL SEQUENCE

<220>
<221> Source
<222> (1)..(66)
<223> /note="Description for artificial sequence: synthetic peptide"

<400> 4

```
Ala Lys Lys Tyr Ala Lys Lys Glu Lys Ala Tyr Ala Lys Ala Lys Lys
1               5               10              15

Ala Glu Ala Lys Ala Ala Lys Lys Ala Lys Ala Glu Ala Lys Lys Tyr
            20              25              30

Ala Lys Ala Ala Lys Ala Glu Lys Lys Glu Tyr Ala Ala Ala Glu Ala
        35              40              45

Lys Tyr Lys Ala Glu Ala Ala Lys Ala Ala Ala Lys Glu Ala Ala Tyr
        50              55              60

Glu Ala
65
```

<210> 5
<211> 77
<212> PRT
<213> ARTIFICIAL SEQUENCE

<220>
<221> Source
<222> (1)..(77)
<223> /note="Description of artificial sequence: synthetic peptide"

<400> 5

```
Ala Lys Lys Tyr Ala Lys Lys Glu Lys Ala Tyr Ala Lys Lys Ala Glu
1               5                   10                  15

Lys Ala Ala Lys Lys Ala Glu Ala Lys Ala Tyr Lys Ala Ala Glu Ala
            20                  25                  30

Lys Lys Lys Ala Lys Ala Glu Ala Lys Lys Tyr Ala Lys Ala Ala Lys
        35                  40                  45

Ala Glu Lys Lys Glu Tyr Ala Ala Ala Glu Ala Lys Tyr Lys Ala Glu
        50                  55                  60

Ala Ala Lys Ala Ala Ala Lys Glu Ala Ala Tyr Glu Ala
65                  70                  75
```

<210> 6
<211> 86
<212> PRT
<213> ARTIFICIAL SEQUENCE

<220>
<221> Source
<222> (1)..(86)
<223> /note="Description of artificial sequence: synthetic peptide"

<400> 6

```
Ala Lys Lys Tyr Ala Lys Lys Glu Lys Ala Tyr Ala Lys Lys Ala Glu
1               5                   10                  15

Lys Ala Ala Lys Lys Ala Glu Ala Lys Ala Tyr Lys Ala Ala Glu Ala
            20                  25                  30

Lys Lys Lys Ala Lys Ala Glu Ala Lys Lys Tyr Ala Lys Ala Ala Lys
        35                  40                  45

Ala Glu Lys Lys Glu Tyr Ala Ala Ala Glu Ala Lys Tyr Lys Ala Glu
        50                  55                  60

Ala Ala Lys Lys Ala Tyr Lys Ala Glu Ala Ala Lys Ala Ala Ala Lys
65                  70                  75                  80

Glu Ala Ala Tyr Glu Ala
                85
```

<210> 7
<211> 109
<212> PRT
<213> ARTIFICIAL SEQUENCE

<220>
<221> Source
<222> (1)..(109)
<223> /note="Description of artificial sequence: synthetic peptide"

<400> 7

```
Ala Lys Lys Tyr Ala Lys Lys Ala Glu Lys Ala Tyr Ala Lys Lys Ala
1               5               10          15

Lys Ala Ala Lys Glu Lys Lys Ala Tyr Ala Lys Lys Glu Ala Lys Ala
        20              25              30

Tyr Lys Ala Ala Glu Ala Lys Lys Lys Ala Lys Ala Glu Ala Lys Lys
    35              40              45

Tyr Ala Lys Glu Ala Ala Lys Ala Lys Lys Glu Ala Tyr Lys Ala Glu
    50              55              60

Ala Lys Lys Tyr Ala Lys Ala Ala Lys Ala Glu Lys Lys Glu Tyr Ala
65              70              75              80

Ala Ala Glu Ala Lys Lys Ala Glu Ala Ala Lys Ala Tyr Lys Ala Glu
                85              90              95

Ala Ala Lys Ala Ala Ala Lys Glu Ala Ala Tyr Glu Ala
            100             105
```